# EUROPÄISCHE PATENTSCHRIFT

(11) **EP 4 478 031 B1**
(45) Veröffentlichungstag und Bekanntmachung des Hinweises auf die Patenterteilung: **21.10.2026**
(21) Anmeldenummer: 24180636.3
(22) Anmeldetag: 07.06.2024
(51) Int. Cl.: G01N 21/3504, G01N 21/27

(54) **VERFAHREN UND VORRICHTUNG ZUM ÜBERPRÜFEN EINES GASDETEKTIONSGERÄTS**
METHOD AND DEVICE FOR CHECKING A GAS DETECTION DEVICE
PROCÉDÉ ET DISPOSITIF DE CONTRÔLE D'UN APPAREIL DE DÉTECTION DE GAZ

(30) Priorität: 14.06.2023 DE 102023115474
(43) Veröffentlichungstag der Anmeldung: 18.12.2024
(73) Patentinhaber: Dräger Safety AG & Co. KGaA, 23560 Lübeck (DE)
(72) Erfinder: Draack, Sebastian, 23558 Lübeck (DE)

(56) Entgegenhaltungen:
- EP-B1- 3 332 230
- DD-A7- 300 325
- DE-A1- 102018 201 947
- DE-C2- 4 111 187
- US-A1- 2013 250 301

## Beschreibung

Die Erfindung betrifft ein Verfahren und eine Vorrichtung, um automatisch ein Gasdetektionsgerät zu überprüfen. Ein solches Gasdetektionsgerät vermag einen räumlichen Bereich auf das Vorhandensein mindestens eines zu detektierenden Zielgases zu überprüfen und / oder die Konzentration eines solchen Zielgases zu ermitteln.

Bekannt geworden sind Gasdetektionsgeräte, welche eine Strahlungsquelle und eine Messkammer umfassen. Eine zu untersuchende Gasprobe gelangt in die Messkammer. Strahlung, welche von der Strahlungsquelle emittiert wird, durchdringt die Messkammer. Ein zu detektierendes Zielgas absorbiert einen Teil der Strahlung, welche die Messkammer durchdringt. Ein Detektor in oder an der Messkammer misst ein Maß für die Intensität von auftreffender Strahlung. Diese Intensität ist ein Maß für die Zielgas-Konzentration. Auch ein Gasdetektionsgerät, welches sich erfindungsgemäß überprüfen lässt, verwendet in einer Ausgestaltung der Erfindung dieses Prinzip.

Bekannt geworden sind auch Gasdetektionsgerate, in welchen ein Referenzsignal zur Kompensation der Störeinfl0sse verwendet wird, wie sie in DE 41 11 187 C2 oder in DE 10 2018 201947 A1 beschrieben werden.

Ein solches Zielgas kann einen Menschen gesundheitlich gefährden. Daher ist es zwingend erforderlich, dass das Gasdetektionsgerät ein schädliches Zielgas zuverlässig detektiert. Andererseits wird gewünscht, dass das Gasdetektionsgerät nur relativ wenige Fehlalarme generiert. Aus beiden Gründen ist es von Zeit zu Zeit erforderlich, das Gasdetektionsgerät zu überprüfen.

Der Erfindung liegt die Aufgabe zugrunde, ein Überprüfungs-Verfahren und eine Überprüfungs-Vorrichtung bereitzustellen, welche es ermöglichen, mit weniger Aufwand als bekannte Verfahren und Vorrichtungen ein Gasdetektionsgerät mit einer Strahlungsquelle, einer Messkammer und einem Detektor zu überprüfen.

Die Aufgabe wird durch ein Überprüfungs-Verfahren mit den Merkmalen des Anspruchs 1 und durch eine Überprüfungs-Vorrichtung mit den Merkmalen des Anspruchs 11 gelöst. Vorteilhafte Ausgestaltungen des erfindungsgemäßen Überprüfungs-Verfahrens sind, soweit sinnvoll, auch vorteilhafte Ausgestaltungen der erfindungsgemäßen Überprüfungs-Vorrichtung und umgekehrt.

Das zu überprüfende Gasdetektionsgerät umfasst eine Messkammer. Diese Messkammer vermag eine zu untersuchende Gasprobe aufzunehmen. Bei einem Einsatz stammt die Gasprobe aus einem zu überwachenden räumlichen Bereich. Weiterhin umfasst das zu überprüfende Gasdetektionsgerät eine Strahlungsquelle und einen Detektor. Die Strahlungsquelle vermag Strahlung in die Messkammer hinein zu emittieren. Diese Strahlung ist insbesondere elektromagnetische Strahlung, z.B. Infrarotstrahlung, oder Schall (akustische Strahlung, insbesondere Ultraschall). Die Strahlung durchdringt mindestens einmal die Messkammer, optional mehrmals.

Der Detektor vermag abhängig von der Intensität von auftreffender Strahlung ein Ausgangssignal zu erzeugen. Das Ausgangssignal bezieht sich auf die Intensität der Strahlung, nachdem die Strahlung mindestens einmal wenigstens einen Teil einer Gasprobe in der Messkammer durchdrungen hat. Die Strahlungs-Intensität wird daher durch ein zu detektierendes Zielgas verändert. Das vom Detektor erzeugte Ausgangssignal korreliert deshalb mit der Konzentration eines Zielgases in einer Gasprobe, wobei die Gasprobe von der Messkammer aufgenommen ist.

Durch das erfindungsgemäße Überprüfungs-Verfahren wird ein derartiges Gasdetektionsgerät automatisch überprüft. Die erfindungsgemäße Überprüfungs-Vorrichtung vermag ein derartiges Gasdetektionsgerät zu überprüfen.

Vorausgesetzt wird, dass das Gasdetektionsgerät in einem Referenz-Zeitraum intakt ist. Wenigstens sind die Strahlungsquelle, die Messkammer und der Detektor intakt, bevorzugt auch eine optionale Auswerteeinheit und eine optionale eigene Spannungsversorgungseinheit. Überprüft wird, ob das Gasdetektionsgerät auch in einem Überprüfungs-Zeitraum intakt ist. In der Regel liegt der Überprüfungs-Zeitraum nach dem Referenz-Zeitraum. Möglich ist aber auch, dass der Referenz-Zeitraum nach dem Überprüfungs-Zeitraum liegt und das Gasdetektionsgerät im Nachhinein überprüft werden soll.

Sowohl im Referenz-Zeitraum als auch im Überprüfungs-Zeitraum werden jeweils die folgenden Schritte durchgeführt:
- In der Messkammer befindet sich eine Gasprobe.
- Die Strahlungsquelle emittiert Strahlung in die Messkammer hinein.
- Ein Maß für die Intensität der emittierten Strahlung wird vorgegeben oder gemessen. Dieses Maß bezieht sich auf die Intensität vor dem Eintritt der Strahlung in die Messkammer und wird als ein Eingangssignal verwendet. Das Eingangssignal kann zeitlich konstant sein oder zeitlich variieren.
- Die emittierte Strahlung durchdringt mindestens einmal wenigstens einen Teil einer Gasprobe in der Messkammer, optional mehrmals, um den optischen Weg zu verlängern. Die Intensität der Strahlung nach dem Durchdringen der Gasprobe hängt vom Eingangssignal und von der Gasprobe in der Messkammer ab.
- Der Detektor generiert ein Ausgangssignal. Dieses Ausgangssignal hängt von der Intensität der Strahlung und somit vom Eingangssignal ab. Das Ausgangssignal bezieht sich auf die Intensität der Strahlung, nachdem die Strahlung mindestens einmal wenigstens einen Teil der Gasprobe in der Messkammer durchdrungen hat.
- Ein Maß für das Systemverhalten eines gedachten Systems wird berechnet, und zwar im Zeitbereich oder im Frequenzbereich. Dieses gedachte System wird mit dem Eingangssignal angeregt und erzeugt als Reaktion auf diese Anregung das Ausgangssignal.

Als ein Maß für ein Referenz-Systemverhalten wird das Maß für das Systemverhalten desjenigen gedachten Systems berechnet, das im Referenz-Zeitraum mit dem vorgegebenen oder gemessenen Eingangssignal angeregt wird und als Reaktion auf diese Anregung das gemessene Ausgangssignal erzeugt. Das Referenz-Systemverhalten bezieht sich also auf ein intaktes Gasdetektionsgerät. Entsprechend wird als ein Maß für ein Überprüfungs-Systemverhalten das Maß für das Systemverhalten desjenigen gedachten Systems berechnet, das im Überprüfungs-Zeitraum mit dem vorgegebenen oder gemessenen Eingangssignal angeregt wird und als Reaktion auf diese Anregung das gemessene Ausgangssignal erzeugt. Wie bereits erwähnt, beschreibt das Eingangssignal die Intensität der emittierten Strahlung, bevor diese Strahlung die Messkammer erreicht, und das Ausgangssignal die Intensität der Strahlung, nachdem die Strahlung die Gasprobe durchdrungen hat. Bevorzugt bezieht sich das Ausgangssignal auf die Intensität der Strahlung, nachdem diese Strahlung mindestens einmal die Messkammer vollständig durchdrungen hat.

Erfindungsgemäß werden die beiden Maße miteinander verglichen, also das Überprüfungs-Systemverhaltens-Maß wird mit dem Referenz-Systemverhaltens-Maß verglichen. Abhängig vom Ergebnis des Vergleichs wird eine Zustandsinformation über den Zustand des Gasdetektionsgeräts im Überprüfungs-Zeitraum hergeleitet.

Die Erfindung lässt sich jedes Mal erneut dann anwenden, wenn das Gasdetektionsgerät überprüft werden soll. In vielen Fällen erspart die Erfindung die Notwendigkeit, das Gasdetektionsgerät nach einem vorgegebenen Zeitraum unabhängig von seinem Zustand außer Betrieb nehmen zu müssen.

Die Erfindung ermöglicht es in vielen Fällen, sowohl einen Fehler der Strahlungsquelle als auch einen Fehler des Detektors zu entdecken. Genauer gesagt: Das Ereignis lässt sich entdecken, dass die Strahlungsquelle und / oder der Detektor im Überprüfungs-Zeitraum sich in relevanter Weise anders verhält als im Referenz-Zeitraum. Insbesondere dann lässt sich ein Fehler der Strahlungsquelle detektieren, wenn ein vorgegebenes Eingangssignal verwendet wird und die Strahlungsquelle diesem Eingangssignal folgen soll. Bei einer fehlerhaften Strahlungsquelle weicht in der Regel die tatsächliche Intensität der Strahlung vom Eingangssignal erheblich ab. Auch ein Fehler der Messkammer lässt sich detektieren, beispielsweise eine Kondensation von Flüssigkeit auf einer Wand der Messkammer. Bevorzugt lassen sich auch ein Fehler einer optionalen Auswerteeinheit und ein Fehler einer optionalen eigenen Spannungsversorgungseinheit des Gasdetektionsgeräts detektieren. Sowohl ein Fehler, der plötzlich auftritt, beispielsweise aufgrund eines externen Einflusses, als ein Fehler, der allmählich aufgrund einer Alterung oder eines Parameterdrifts auftritt, lassen sich dank der Erfindung entdecken. Dieser Vorteil wird erzielt, weil in vielen Fällen sowohl eine fehlerhafte Strahlungsquelle als auch ein fehlerhafter Detektor als auch eine fehlerhafte Messkammer zu einem signifikant veränderten Systemverhalten und damit auch zu einem signifikant veränderten Maß für das Systemverhalten führen. Der Vergleich führt daher zu einer Abweichung zwischen den beiden Maßen für das Systemverhalten, wobei die Abweichung größer als eine vorgegebene Toleranz ist.

Erfindungsgemäß wird das Überprüfungs-Systemverhalten mit dem Referenz-Systemverhalten verglichen. Das Überprüfungs-Systemverhalten beschreibt die Reaktion des gedachten Systems im Überprüfungs-Zeitraum auf eine Anregung durch die Strahlungsquelle, das Referenz-Systemverhalten die Reaktion desselben gedachten Systems im Referenz-Zeitraum auf dieselbe Anregung. In diesen beiden Vorgängen wird dasselbe Gerät untersucht. Die Begriffe "dieselbe Anregung" und "dasselbe gedachte System" treffen natürlich nur dann zu, wenn das Gasdetektionsgerät auch im Überprüfungs-Zeitraum intakt ist. Mit anderen Worten: Das Überprüfungs-Systemverhalten beschreibt den Zustand des Gasdetektionsgeräts im Überprüfungs-Zeitraum, das Referenz-Systemverhalten beschreibt den Zustand des Gasdetektionsgeräts im Referenz-Zeitraum. Insbesondere werden in beiden Zeiträumen dasselbe Gerät und somit dieselbe Strahlungsquelle, dieselbe Messkammer und derselbe Detektor verwendet. Falls die Strahlungsquelle, die Messkammer und der Detektor auch im Überprüfungs-Zeitraum intakt sind, so stimmt idealerweise das Überprüfungs-Systemverhalten mit dem Referenz-Systemverhalten überein. Bevorzugt wird eine Toleranz vorgegeben, und die Strahlungsquelle, die Messkammer und der Detektor werden dann als intakt angesehen, wenn die beiden Systemverhaltens-Maße um nicht mehr als die Toleranz voneinander abweichen. Diese Toleranz berücksichtigt einen unvermeidlichen Messfehler sowie mögliches Messrauschen und Prozessrauschen.

Das Referenz-Systemverhalten wird bei einem Zustand erfasst, bei dem das Gasdetektionsgerät - oder wenigstens die Strahlungsquelle, die Messkammer und der Detektor - intakt ist. In vielen Fällen ist es ausreichend, dass folgende beiden Voraussetzungen erfüllt sind:
- Im Referenz-Zeitraum und im Überprüfungs-Zeitraum werden zwei Gasproben verwendet, welche die durchdringende Strahlung ausreichend übereinstimmend absorbieren. Dies ist in der Regel insbesondere dann der Fall, wenn die beiden Gasproben ausreichend genau chemisch übereinstimmen.
- Das Eingangssignal, also die emittierte Strahlung, ist ausreichend breitbandig, so dass auch ein Fehler des Geräts detektiert werden kann, welcher nur in einem Teilbereich des Frequenzbands auftritt.

Oft ist es dank der Erfindung nicht erforderlich sicherzustellen, dass weitere Voraussetzungen erfüllt sind, insbesondere dass die im Referenz-Zeitraum verwendete Gasprobe eine bestimmte chemische Zusammensetzung aufweist. Weiterhin ist es häufig nicht erforderlich, die Strahlungsquelle im Überprüfungs-Zeitraum und im Referenz-Zeitraum mit dem gleichen zeitlichen Verlauf anzusteuern. Insbesondere ist es nicht erforderlich, dass die jeweilige elektrische Spannung, die an der Strahlungsquelle anliegt, in beiden Zeiträumen übereinstimmt. Dieser Vorteil wird erzielt, weil erfindungsgemäß zwei Systemverhalten von zwei gedachten Systemen berechnet und miteinander verglichen werden und diese beiden Systemverhalten bei intakten Bestandteilen des Gasdetektionsgeräts auch dann ausreichend genau übereinstimmen, wenn in den beiden Zeiträumen unterschiedliche Ansteuerungen und daher unterschiedliche Eingangssignale verwendet werden.

Das Überprüfungs-Systemverhalten stimmt mindestens dann bis auf eine unvermeidliche Toleranz mit dem Referenz-Systemverhalten überein, wenn folgende Bedingungen ausreichend genau erfüllt sind:
- Das Gasdetektionsgerät ist auch im Überprüfungs-Zeitraum intakt.
- Die Gasprobe, die sich im Überprüfungs-Zeitraum in der Messkammer befindet, stimmt ausreichend genau mit der Gasprobe überein, die sich im Referenz-Zeitraum in der Messkammer befindet.
- In beiden Zeiträumen wirken annähernd die gleichen Umgebungsbedingungen auf die Messkammer ein.

Bevorzugt wird ein Alarm generiert, wenn die beiden Systemverhaltens-Maße stärker als eine vorgegebene Toleranz voneinander abweichen. Eine Ursache für eine Abweichung ist ein Defekt eines Bestandteils des Gasdetektionsgeräts. Dieser Defekt kann schlagartig aufgetreten sein, beispielsweise durch einen Ausfall, oder allmählich, beispielsweise durch den Drift eines Parameters, insbesondere durch eine Alterung. Bevorzugt lässt sich ein Alarm als Anlass dafür verwenden, das Gasdetektionsgerät genauer zu untersuchen.

Die Erfindung setzt nicht voraus, dass in beiden Zeiträumen die gleiche Gasprobe verwendet wird und die gleichen Umgebungsbedingungen auf die Messkammer einwirken. Selbst wenn die Gasproben oder die Umgebungsbedingungen sich voneinander unterscheiden, wird möglicherweise das Gasdetektionsgerät zu Unrecht als defekt eingestuft. Auch in diesem Fall wird aber in der Regel ein tatsächlich defektes Gasdetektionsgerät als defekt erkannt.

Ein einstellbarer Parameter ist die Toleranz. Falls die beiden Systemverhaltens-Maße mehr als diese Toleranz voneinander abweichen, so wird das Gasdetektionsgerät als defekt eingestuft. Möglich ist, dass diese Toleranz abhängig von möglichen Veränderungen der Umgebungsbedingungen vorgegeben wird. Diese Ausgestaltung reduziert die Gefahr, dass eine stark veränderte Umgebungsbedingung dazu führt, dass das Gasdetektionsgerät zu Unrecht als defekt eingestuft wird.

Im Gegensatz zu vielen bekannten Verfahren zur Überprüfung setzt die Erfindung nicht voraus, dass eine Gasprobe, die im Referenz-Zeitraum verwendet wird, eine vorab bekannte Zusammensetzung aufweist.

Die Erfindung setzt nicht voraus, dass das Eingangssignal im Überprüfungs-Zeitraum genau mit dem Eingangssignal im Referenz-Zeitraum übereinstimmt. Die Erfindung setzt auch nicht zwingend voraus, dass vorab ermittelt wird, wie ein mögliches Störsignal auf ein Signal des Detektors einwirkt und / oder in einem Signal des Detektors enthalten ist. Diese Wirkungen werden erzielt, weil zwei Maße für ein Systemverhalten miteinander verglichen werden
Erfindungsgemäß erzeugt der Detektor in beiden Zeiträumen jeweils ein Ausgangssignal. Dieses Ausgangssignal ist ein Maß für die Intensität der Strahlung, nachdem die Strahlung eine Gasprobe in der Messkammer durchdrungen hat. Verschiedene Ausgestaltungen sind möglich, welches Maß der Detektor in den beiden Zeiträumen jeweils misst.

In einer Ausgestaltung trifft die emittierte Strahlung auf den Detektor auf, nachdem die Strahlung mindestens einmal die Messkammer vollständig durchdrungen hat. Der Detektor ist als ein Photodetektor ausgestaltet und erzeugt das Ausgangssignal abhängig von der Intensität, mit der die Strahlung auf den Photodetektor auftrifft. Bei dieser Ausgestaltung ist das Ausgangssignal relativ unempfindlich gegenüber Umgebungsbedingungen wie Temperatur oder Druck oder Feuchte oder auch gegenüber Vibrationen.

In einer anderen Ausgestaltung löst die emittierte Strahlung in der Messkammer einen physikalischen Effekt aus. Beispielsweise verändert die Strahlung die Temperatur und / oder den Druck in der Messkammer oder erzeugt einen akustischen Effekt. Der Detektor misst ein Maß für den erzeugten physikalischen Effekt. Diese Ausgestaltung ist in vielen Fällen relativ unempfindlich gegenüber der Lichtstärke in der Umgebung.

Erfindungsgemäß wird in beiden Zeiträumen jeweils ein Maß für das Systemverhalten des gedachten Systems berechnet. In einer bevorzugten Ausgestaltung umfasst der Schritt, das Maß für das Systemverhalten zu berechnen, in beiden Zeiträumen jeweils den folgenden Schritt: Ein Maß für die Kreuzkorrelation zwischen dem Eingangssignal und dem Ausgangssignal wird berechnet.

Das Eingangssignal und das Ausgangssignal sind zeitaufgelöste Signale, also Signale im Zeitbereich. In der Regel wird daher auch die Kreuzkorrelation im Zeitbereich berechnet. In einer Fortbildung dieser Ausgestaltung wird für beide Zeiträume die jeweilige Kreuzkorrelation aus dem Zeitbereich in den Frequenzbereich transformiert, insbesondere durch eine Fourier-Transformation oder eine sonstige Laplace-Transformation. Durch die Transformation wird bevorzugt die jeweilige Übertragungsfunktion im Komplexen des gedachten Systems berechnet. Eine Referenz-Übertragungsfunktion beschreibt das Systemverhalten im Referenz-Zeitraum, eine Überprüfungs-Übertragungsfunktion des Systemverhalten im Überprüfungs-Zeitraum. Die Übertragungsfunktion im Komplexen lässt sich manchmal auch ohne Verwendung einer Kreuzkorrelation berechnen.

**In** einer Ausgestaltung wird das erfindungsgemäße Überprüfungs-Verfahren unter Verwendung einer Signalverarbeitungseinheit durchgeführt. Diese Signalverarbeitungseinheit berechnet sowohl im Referenz-Zeitraum als auch im Überprüfungs-Zeitraum das jeweilige Maß für das Systemverhalten, vergleicht diese miteinander und liefert die Zustands-Information über das Gasdetektionsgerät.

**In** einer Realisierungsform ist diese Signalverarbeitungseinheit ein Bestandteil des zu überprüfenden Gasdetektionsgeräts. Das Gasdetektionsgerät vermag sich also selber zu überprüfen. **In** einer anderen Ausgestaltung ist die Signalverarbeitungseinheit ein Bestandteil einer Rechnereinheit. Diese Rechnereinheit ist räumlich vom Gasdetektionsgerät entfernt. Zwischen dem Gasdetektionsgerät und der Rechnereinheit ist wenigstens zeitweise eine Datenverbindung hergestellt. Diese Ausgestaltung erleichtert es, eine leistungsfähige Signalverarbeitungseinheit vorzusehen. Dies ist insbesondere dann von Vorteil, wenn das Gasdetektionsgerät ein tragbares Gerät ist, insbesondere ein tragbares Gerät, welches ein Benutzer mit sich führt. Die Rechnereinheit kann als ein stationäres Gerät ausgestaltet sein.

Erfindungsgemäß umfasst das Gasdetektionsgerät eine Messkammer und einen Detektor. **In** einer Ausgestaltung umfasst das Gasdetektionsgerät zusätzlich eine Referenzkammer und einen Referenz-Detektor. Während die Messkammer wenigstens im Überprüfungs-Zeitraum mit einer Gasprobe gefüllt ist, die das oder mindestens ein zu detektierendes Zielgas aufweisen kann, ist die Referenzkammer mit einem Referenzgas gefühlt, das idealerweise frei von dem oder jedem zu detektierenden Zielgas ist. Bevorzugt ist die Referenzkammer fluiddicht gegen die Messkammer abgedichtet. Die beiden Kammern sind bezüglich der emittierten Strahlung parallel angeordnet. Daher durchdringt die Strahlung, welche die Strahlungsquelle emittiert, sowohl die Messkammer als auch die Referenzkammer jeweils mindestens einmal. Die Strahlung durchdringt also die Gasprobe in der Messkammer und das Referenzgas in der Referenzkammer. Dies gilt sowohl für den Referenz-Zeitraum als auch für den Überprüfungs-Zeitraum.

Erfindungsgemäß erzeugt der Detektor abhängig von der Intensität von emittierter Strahlung in der Messkammer ein zeitaufgelöstes Signal, nämlich das Ausgangssignal. Diese Strahlung hat bevorzugt die Messkammer mindestens einmal vollständig durchdrungen. Gemäß der gerade beschriebenen Ausgestaltung erzeugt der Referenz-Detektor abhängig von emittierter Strahlung in der Referenzkammer ein zeitaufgelöstes Referenzsignal. Diese Strahlung hat bevorzugt die Referenzkammer mindestens einmal vollständig durchdrungen.

Die Ausgestaltung mit dem Ausgangssignal vom Detektor und dem Referenzsignal vom Referenz-Detektor ermöglicht es, diese beiden Signale miteinander zu vergleichen und dadurch ein Zielgas zu detektieren oder das Vorhandensein eines Zielgases auszuschließen. Das oder jedes Zielgas wirkt nur auf die Messkammer, aber idealerweise nicht auf die Referenzkammer. Umgebungsbedingungen, insbesondere die Umgebungstemperatur und die Umgebungsfeuchte, sowie Vibrationen und andere externe Einflüsse wirken hingegen auf beide Kammern.

Erfindungsgemäß wird sowohl im Referenz-Zeitraum als auch im Überprüfungs-Zeitraum jeweils ein Maß für das Systemverhalten eines gedachten Systems berechnet. Dieses gedachte System wird mit dem jeweiligen Eingangssignal angeregt und liefert als Reaktion auf die Anregung das Ausgangssignal. Gemäß der gerade beschriebenen Ausgestaltung wird abweichend als Eingangssignal das Signal verwendet, welches der Referenz-Detektor generiert, nachdem Strahlung mindestens einmal die Referenzkammer durchdrungen hat. Dies gilt sowohl für den Schritt, das Maß für das Referenz-Systemverhalten zu berechnen, als auch für den Schritt, das Maß für das Überprüfungs-Systemverhalten zu berechnen.

Diese Ausgestaltung kompensiert in vielen Fällen bis zu einem gewissen Grad den Einfluss von Umgebungsbedingungen auf das jeweilige Systemverhalten. Oft ist es nicht erforderlich, im Referenz-Zeitraum und im Überprüfungs-Zeitraum genau die gleichen Umgebungsbedingungen herzustellen.

Durch das erfindungsgemäße Überprüfungs-Verfahren und die erfindungsgemäße Überprüfungs-Vorrichtung wird das Gasdetektionsgerät überprüft. In einer Ausgestaltung wird die Erfindung verwendet, um das Gasdetektionsgerät zu justieren und / oder zu kalibrieren. Gemäß dieser Ausgestaltung hängt das Ausgangssignal von der Intensität der Strahlung in der Messkammer ab, bevorzugt von der Intensität, nachdem diese Strahlung die Messkammer durchdrungen hat, und zusätzlich von dem verwendeten Wert eines Parameters. Das gedachte System, das mit dem Eingangssignal angeregt wird und als Reaktion auf die Anregung das Ausgangssignal liefert, hängt zusätzlich vom verwendeten Parameter-Wert ab. Beispielsweise umfasst das Ausgangssignal ein Maß für die Konzentration eines zu detektierenden Gases, wobei diese Konzentration zeitlich veränderlich sein kann. Der Parameter beschreibt einen funktionalen Zusammenhang zwischen einem Ausgangssignal des Detektors und der Zielgas-Konzentration und ist beispielsweise ein Proportionalitätsfaktor.

Erfindungsgemäß wird im Referenz-Zeitraum das Referenz-Systemverhalten berechnet. Gemäß der Ausgestaltung mit dem Parameter werden mehrere mögliche Werte für die Parameter vorgegeben. Jeder mögliche Parameter-Wert führt zu jeweils einem gedachten System. Für jeden möglichen Parameter-Wert wird im Überprüfungs-Zeitraum das jeweilige Überprüfungs-Systemverhalten dieses gedachten Systems berechnet und jeweils mit dem vorab berechneten Referenz-Systemverhalten verglichen. Für jeden möglichen Parameter-Wert wird dadurch ein Vergleichsergebnis berechnet. Welcher Wert tatsächlich für den Parameter verwendet wird, wird abhängig von diesem Vergleichsergebnis festgelegt. Beispielsweise wird derjenige Wert für den Parameter verwendet, der zu der geringsten Abweichung zwischen dem Referenz-Systemverhalten und dem Überprüfungs-Systemverhalten führt.

In vielen Fällen erleichtert diese Ausgestaltung es, das Gasdetektionsgerät zu justieren. Oft ist es nicht erforderlich, eine Justier-Gasprobe mit einer bestimmten ausreichend genau bekannten chemischen Zusammensetzung zu verwenden, wobei das zu justierende Gasdetektionsgerät diese Justier-Gasprobe analysiert. Diese Ausgestaltung lässt sich aber auch in Verbindung mit einer Justier-Gasprobe anwenden.

Die Erfindung betrifft weiterhin ein Mess-Verfahren und eine Mess-Anordnung, um die Konzentration mindestens eines Zielgases zu messen. Das Verfahren wird unter Verwendung eines Gasdetektionsgeräts durchgeführt, und die Anordnung umfasst ein Gasdetektionsgerät, welches so wie gerade beschrieben aufgebaut ist, also eine Messkammer, einen Detektor und eine Strahlungsquelle umfasst. Für dieses Gasdetektionsgerät wird gemäß einer Ausgestaltung mindestens einmal ein erfindungsgemäßes Überprüfungs-Verfahren durchgeführt. Die Mess-Anordnung umfasst eine erfindungsgemäße Überprüfungs-Vorrichtung, welche dazu ausgestaltet ist, das Gasdetektionsgerät zu überprüfen.

Im Folgenden wird die Erfindung anhand eines Ausführungsbeispiels beschrieben. Hierbei zeigt
- Figur 1: den Transmissionsgrad von Methan in Abhängigkeit von der Wellenlänge;
- Figur 2: den Transmissionsgrad von Propan in Abhängigkeit von der Wellenlänge;
- Figur 3: schematisch das Gasdetektionsgerät im Referenz-Zeitraum;
- Figur 4: schematisch die Signalverarbeitungseinheit von Figur 3 im Detail;
- Figur 5: schematisch das Gasdetektionsgerät im Überprüfungs-Zeitraum;
- Figur 6: schematisch die Signalverarbeitungseinheit von Figur 5 im Detail;
- Figur 7: eine abweichende Ausgestaltung des Gasdetektionsgeräts mit einer Messkammer und zusätzlich einer Referenzkammer im Referenz-Zeitraum;
- Figur 8: die Ausgestaltung von Figur 7 im Überprüfungs-Zeitraum;
- Figur 9: eine abweichende Ausgestaltung, wobei die Signalverarbeitungseinheit ein Bestandteil einer räumlich beabstandeten Rechnereinheit ist.

Im Ausführungsbeispiel wird die Erfindung eingesetzt, um ein Gasdetektionsgerät zu überprüfen. Dieses Gasdetektionsgerät wird dafür verwendet oder lässt sich dafür verwenden, um einen räumlichen Bereich auf mehrere verschiedene brennbare Zielgase zu untersuchen. In einer Ausgestaltung wird die jeweilige Konzentration jedes Zielgases ermittelt, und bevorzugt wird eine Information über mindestens eine ermittelte Zielgas-Konzentration in einer von einem Menschen wahrnehmbaren Form ausgegeben. In einer anderen Ausgestaltung wird ein Alarm ausgegeben, wenn die gemessene Konzentration mindestens eines Zielgases oberhalb einer vorgegebenen Schranke liegt.

Das zu überprüfende Gasdetektionsgerät nutzt ein aus dem Stand der Technik bekanntes Prinzip aus, nämlich das folgende: Verschiedene Zielgase schwächen elektromagnetische und / oder akustische Strahlung ab. Ein Maß für die Abschwächung ist der spektrale Verlauf, ausgedrückt als Transmissionsgrad Tr in %, in Abhängigkeit von der Wellenlänge λ von Strahlung. Je geringer der Transmissionsgrad Tr[λ] ist, desto stärker schwächt das Zielgas die Strahlung ab.

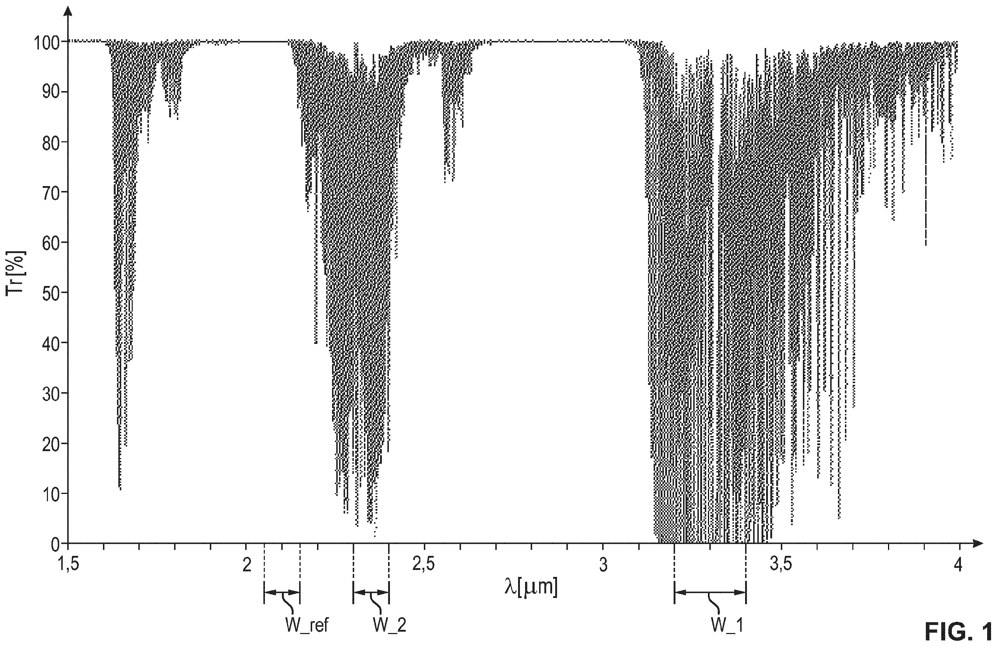

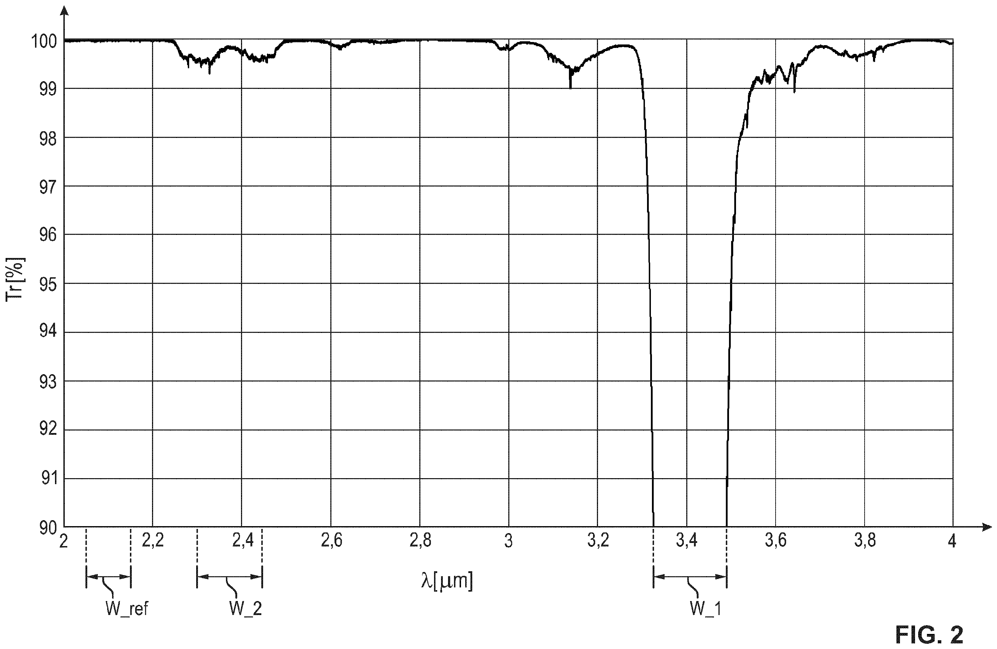

Figur 1 zeigt beispielhaft den spektralen Verlauf für den Kohlenwasserstoff Methan, Figur 2 den für Propan, und zwar jeweils bei einer beispielhaften Konzentration von Methan bzw. Propan. In diesem Beispiel durchdringt elektromagnetische Strahlung eine Gasprobe in einer Messkammer. Auf der x-Achse ist die Wellenlänge λ in Mikrometer [µm] aufgetragen, auf der y-Achse der Transmissionsgrad Tr in Prozent [%].

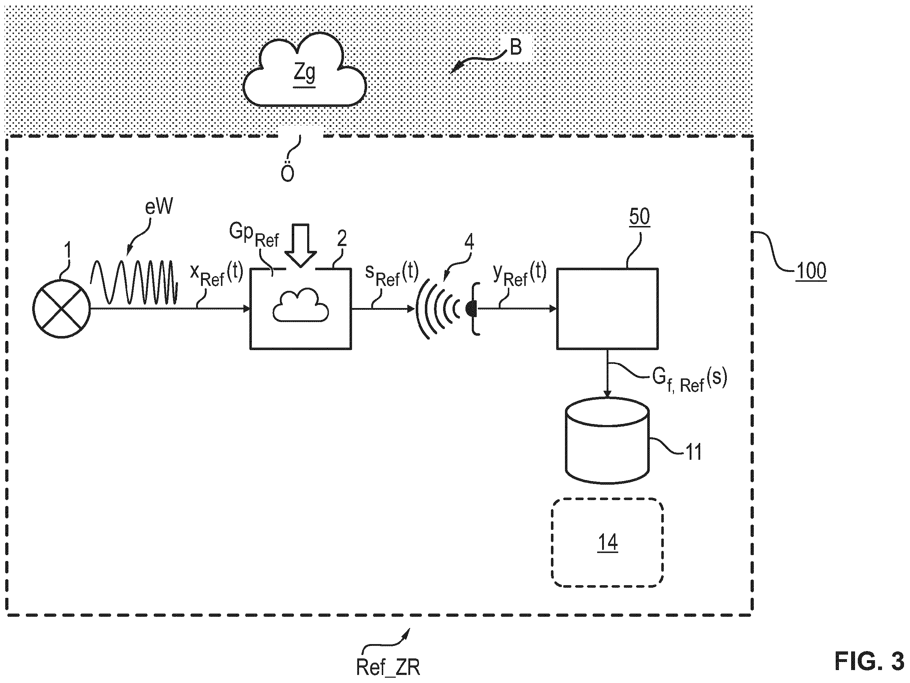

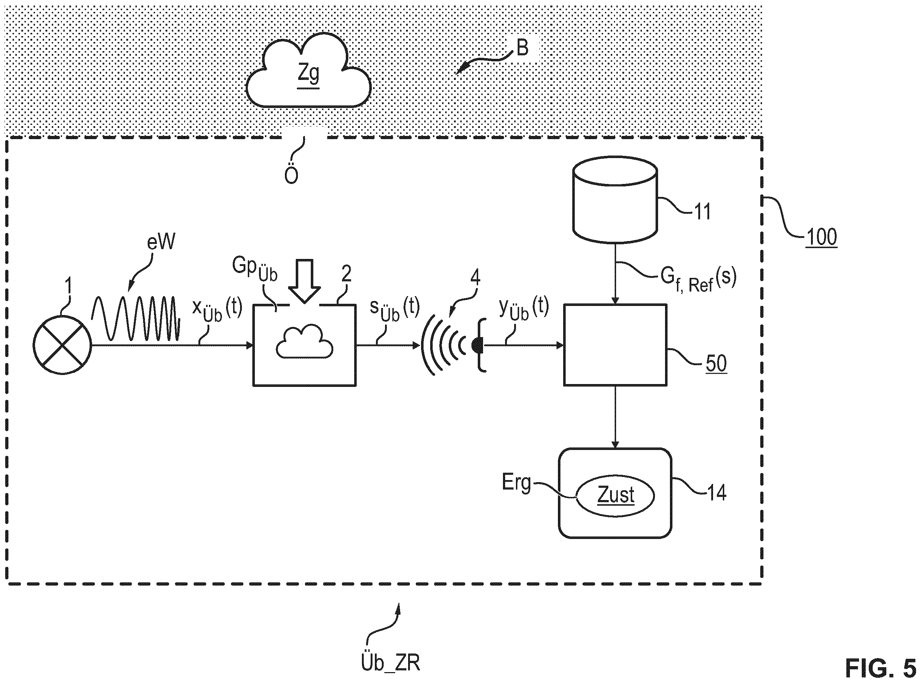

Figur 3 und Figur 5 zeigen schematisch das Gasdetektionsgerät 100 gemäß einer Ausgestaltung des Ausführungsbeispiels. Das Gasdetektionsgerät 100 umfasst eine Messkammer 2 und eine Strahlungsquelle 1.

Die Messkammer 2 steht über eine Öffnung Ö wenigstens zeitweise in einer Fluidverbindung mit dem zu überwachenden räumlichen Bereich B. Dieser Bereich B kann mindestens ein zu detektierendes Zielgas Zg aufweisen. Eine Gasprobe Gp_{Ref}, Gp_{Üb} fließt durch die Öffnung Ö aus dem Bereich B in die Messkammer 2, beispielsweise indem eine Pumpe oder eine sonstige Fluidfördereinheit die Gasprobe Gp_{Ref}, Gp_{Üb} ansaugt und / oder indem die Gasprobe Gp_{Ref}, Gp_{Üb} in die Messkammer 2 diffundiert.

Die Strahlungsquelle 1 emittiert Strahlung eW mit einem Frequenzband. In einer Realisierungsform emittiert die Strahlungsquelle 1 elektromagnetische Strahlung, insbesondere Infrarotstrahlung, in einer anderen Realisierungsform akustische Strahlung (Schall oder Ultraschall). Mit dem nachfolgend verwendeten Begriff "Strahlung" sollen nachfolgend insbesondere elektromagnetische Strahlung im sichtbaren Bereich, Infrarot-Bereich oder Ultraviolett-Bereich sowie Schall und Ultraschall umfasst sein.

Diese Strahlung eW durchdringt mindestens einmal, optional mehrmals die Messkammer 2. Das Gasdetektionsgerät 100 wendet ein Prinzip an, das aus dem Stand der Technik wohlbekannt ist. Das oder mindestens ein zu detektierendes Zielgas Zg in der Messkammer 2 schwächt die Strahlung eW in einem Frequenzbereich ab, was mit Bezug auf Figur 1 und Figur 2 beschrieben wurde. Dieser Frequenzbereich unterscheidet sich in der Regel von Zielgas zu Zielgas und ist vorab bekannt. Die Intensität der jeweiligen Abschwächung hängt von der Zielgas-Konzentration ab. Der Frequenzbereich, in dem die Abschwächung stattfindet, hängt in der Regel nur vom Zielgas-Typ ab, aber nicht von der Zielgas-Konzentration. Ein Detektor misst ein Maß für die Intensität der auftreffenden Strahlung, nachdem die emittierte Strahlung eW die Messkammer 2 durchdrungen hat. Dieses Maß für die Intensität korreliert mit der Zielgas-Konzentration.

In Figur 3 und Figur 5 wird die zeitlich variierende Intensität der emittierten Strahlung eW durch ein Signal x_{Ref}(t), x_{Üb}(t) beschrieben. Dieses Signal x_{Ref}(t), x_{Üb}(t) bezieht sich auf einen Bereich flussaufwärts von der Messkammer 2 und wird nachfolgend als das Eingangssignal bezeichnet. Das Eingangssignal x_{Ref}(t), x_{Üb}(t) wird gemessen oder ist durch die Ansteuerung der Strahlungsquelle 1 vorgegeben. Falls als Eingangssignal eine Ansteuerung der Strahlungsquelle 1 verwendet wird, so kann die tatsächliche Intensität von der Ansteuerung deshalb abweichen, weil die Strahlungsquelle 1 fehlerhaft ist. In manchen Fällen lässt sich dank der Erfindung auch ein Indiz für einen solchen Fehler der Strahlungsquelle 1 entdecken.

Bevorzugt emittiert die Strahlungsquelle 1 die Strahlung eW dergestalt, dass das Eingangssignal x_{Ref}(t), x_{Üb}(t) sinusförmig mit über der Zeit variierender Frequenz ist (frequency sweep signal). In einer Realisierungsform emittiert die Strahlungsquelle 1 die Strahlung eW dergestalt, dass das Eingangssignal x_{Ref}(t), x_{Üb}(t) idealerweise ein weißes Rauschsignal ist. In der Realität lässt sich ein weißes Rauschen nur näherungsweise erreichen. Um einem weißen Rauschsignal nahe zu kommen, wird bevorzugt in der Praxis ein breitbandiges Rauschsignal mit begrenzter Energie emittiert.

Die Strahlung eW durchdringt die Messkammer 2 jeweils mindestens einmal vollständig. Die Intensität der Strahlung eW, welche die Messkammer 2 durchdrungen hat, wird durch das Signal s_{Ref}(t), s_{Üb}(t) beschrieben.

Das Gasdetektionsgerät 100 analysiert die Gasprobe Gp_{Ref}, Gp_{Üb} in der Messkammer 2. Für die Dauer dieser Analyse wird die Gasprobe Gp_{Ref}, Gp_{Üb} als ein lineares zeitinvariantes System behandelt. Diese Annahme ist insbesondere deshalb in der Regel gerechtfertigt, weil sich weder das Gasdetektionsgerät 100 selber noch die chemische Zusammensetzung der Gasprobe Gp_{Ref}, Gp_{Üb} in der Messkammer 2 wesentlich ändert, während die Gasprobe Gp_{Ref}, Gp_{Üb} analysiert wird.

Das Gasdetektionsgerät 100 umfasst weiterhin einen Detektor 4, der in oder an der Messkammer 2 angeordnet ist. Der Detektor 4 wird durch auftreffende Strahlung eW angeregt und liefert ein zeitaufgelöstes Signal. Das Signal x_{Ref}(t), x_{Üb}(t), welches die Intensität der auf die Messkammer 2 auftreffenden Strahlung eW beschreibt, wird als das Eingangssignal für das gerade beschriebene lineare zeitinvariante System behandelt. Das Signal, welches der Detektor 4 als Reaktion auf die Anregung durch das Signal s_{Ref}(t), s_{Üb}(t) liefert, wird als das Ausgangssignal dieses gedachten Systems behandelt und mit y_{Ref}(t), y_{Üb}(t) bezeichnet. Diese beiden Bezeichnungen werden nachfolgend erläutert.

Das Gasdetektionsgerät 100 wird in einem Referenz-Zeitraum Ref_ZR und in einem Überprüfungs-Zeitraum Üb_ZR verwendet. Vorausgesetzt wird, dass das Gasdetektionsgerät 100 im Referenz-Zeitraum Ref_ZR intakt ist. Im Überprüfungs-Zeitraum Üb_ZR soll überprüft werden, ob das Gasdetektionsgerät 100 immer noch intakt ist oder aber einen Fehler aufweist - allgemein: ob sich sein Zustand im Vergleich zum Zustand im Referenz-Zeitraum Ref_ZR in relevanter Weise verändert hat. Möglich ist auch, dass der Referenz-Zeitraum Ref_ZR nach dem Überprüfungs-Zeitraum Üb_ZR liegt und im Nachherein festgestellt werden soll, ob das Gasdetektionsgerät 100 im Überprüfungs-Zeitraum Üb_ZR intakt oder fehlerhaft war.

Insbesondere können folgende Fehler auftreten, welche durch die Erfindung detektiert werden:
- Ein Bauteil fällt plötzlich aus.
- Der Detektor 4 wird fehlerhaft, beispielsweise weil sich Schadgase ablagern oder durch Alterung oder durch einen plötzlichen externen Einfluss.
- Die Strahlungsquelle 1 wird fehlerhaft, beispielsweise ebenfalls durch Ablagerungen oder Alterung oder durch einen plötzlichen Ausfall.
- Eine Wand der Messkammer 2 absorbiert oder streut Strahlung eW, was insbesondere dann ein Fehler ist, wenn die Strahlung die Messkammer 2 mehrmals durchdringen soll, um den optischen Weg zu verlängern. Eine mögliche Ursache ist, dass Wasser auf einer reflektierenden Wand kondensiert.
- Die Spannungsversorgung für die Strahlungsquelle 1 wird schwächer, beispielsweise aufgrund einer Alterung einer internen Spannungsversorgungseinheit oder Fehler in einem Spannungsversorgungsnetz.

Das Ausgangssignal, welches der Detektor 4 im Referenz-Zeitraum Ref_ZR erzielt, wird mit y_{Ref}(t) bezeichnet. Das Ausgangssignal aus dem Überprüfungs-Zeitraum Üb_ZR wird mit y_{Üb} (t) bezeichnet. Das Eingangssignal im Referenz-Zeitraum Ref_ZR wird mit x_{Ref}(t) bezeichnet, das Eingangssignal im Überprüfungs-Zeitraum Üb_ZR mit x_{Üb}(t). Entsprechend wird das Maß für die Intensität der Strahlung eW, die im Referenz-Zeitraum Ref_ZR auf den Detektor 4 auftrifft, mit s_{Ref}(t) bezeichnet und das Maß für die Intensität der im Überprüfungs-Zeitraum Üb_ZR auftreffenden Strahlung eW mit s_{Üb}(t). In Figur 3 bis Figur 9 ist eingetragen, ob die Darstellung sich auf den Referenz-Zeitraum Ref_ZR oder auf den Überprüfungs-Zeitraum Üb_ZR bezieht.

Bei fehlerfreiem Gasdetektionsgerät 100 und übereinstimmenden Eingangssignalen x_{Ref}(t) und x_{Üb}(t) stimmen die beiden Signale s_{Ref}(t) und s_{Üb}(t) und die beiden Ausgangssignale y_{Ref}(t) und y_{Üb}(t)idealerweise überein.

Das Eingangssignal x_{Ref}(t) und das Ausgangssignal y_{Ref}(t) beziehen sich auf denselben Referenz-Zeitraum Ref_ZR und auf dieselben Abtast-Zeitpunkte in diesem übereinstimmenden Referenz-Zeitraum Ref_ZR. Entsprechend beziehen das Eingangssignal x_{Üb}(t) und das Ausgangssignal y_{Üb}(t) sich auf denselben Überprüfungs-Zeitraum Üb_ZR und auf dieselben Abtast-Zeitpunkte in diesem übereinstimmenden Überprüfungs-Zeitraum Üb_ZR.

Im Ausführungsbeispiel werden im Referenz-Zeitraum Ref_ZR und im Überprüfungs-Zeitraum Üb_ZR folgende Bedingungen hergestellt:
- In beiden Zeiträumen Ref_ZR, Üb_ZR emittiert die Strahlungsquelle 1 ein Strahlung eW mit einem Frequenzband, wobei in diesem Frequenzband die Intensität der Strahlung eW oberhalb eines vorgegebenen unteren Schwellwerts liegt.
- Die Gasprobe Gp_{Üb}, die sich im Überprüfungs-Zeitraum Üb_ZR in der Messkammer 2 befindet, absorbiert in diesem Frequenzband die Strahlung eW bis auf eine vorgegebene Toleranz genauso wie die Gasprobe Gp_{Ref} im Referenz-Zeitraum Ref_ZR. Beispielsweise stimmen die beiden Gasproben chemisch weitgehend überein.

Eine Signalverarbeitungseinheit 50 empfängt das Eingangssignal x_{Ref}(t), x_{Üb}(t) und das Ausgangssignal y_{Ref}(t), y_{Üb}(t), verarbeitet diese Signale und liefert ein Ergebnis, welches eine Zustands-Information Zust über den Zustand des Gasdetektionsgeräts 100 umfasst. Diese Zustands-Information Zust bezieht sich auf den Zustand im Überprüfungs-Zeitraum Üb_ZR. In der Ausgestaltung gemäß Figur 3 und Figur 5 ist die Signalverarbeitungseinheit 50 ein Bestandteil des Gasdetektionsgeräts 100, und das Gasdetektionsgerät 100 umfasst weiterhin eine Ausgabeeinheit 14. Die Ausgabeeinheit 14 gibt die Zustands-Information Zust visuell und / oder akustisch und / oder haptisch (durch Vibrationen) aus.

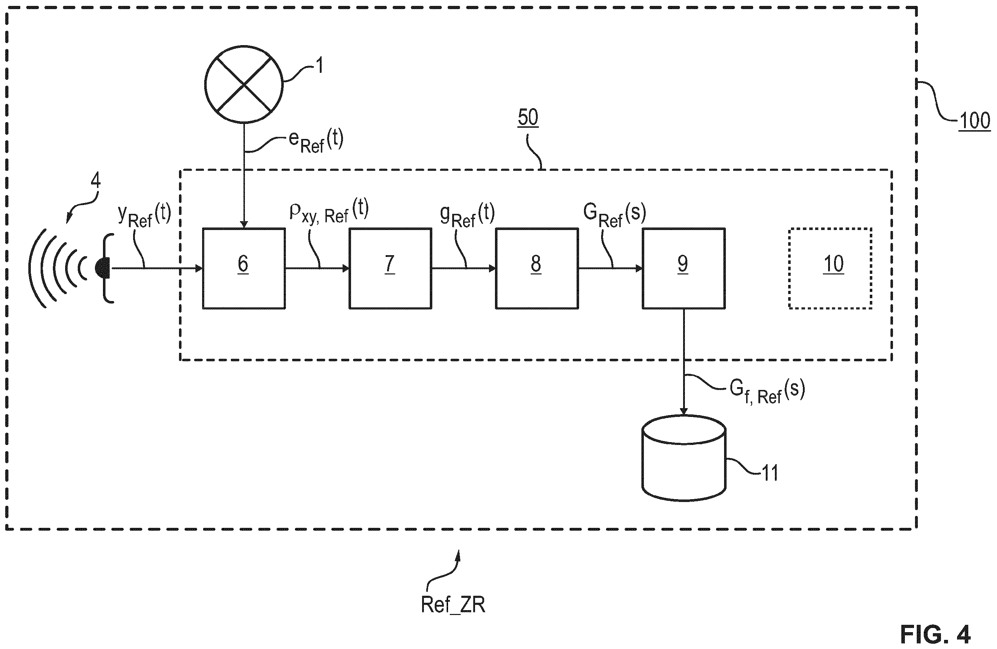

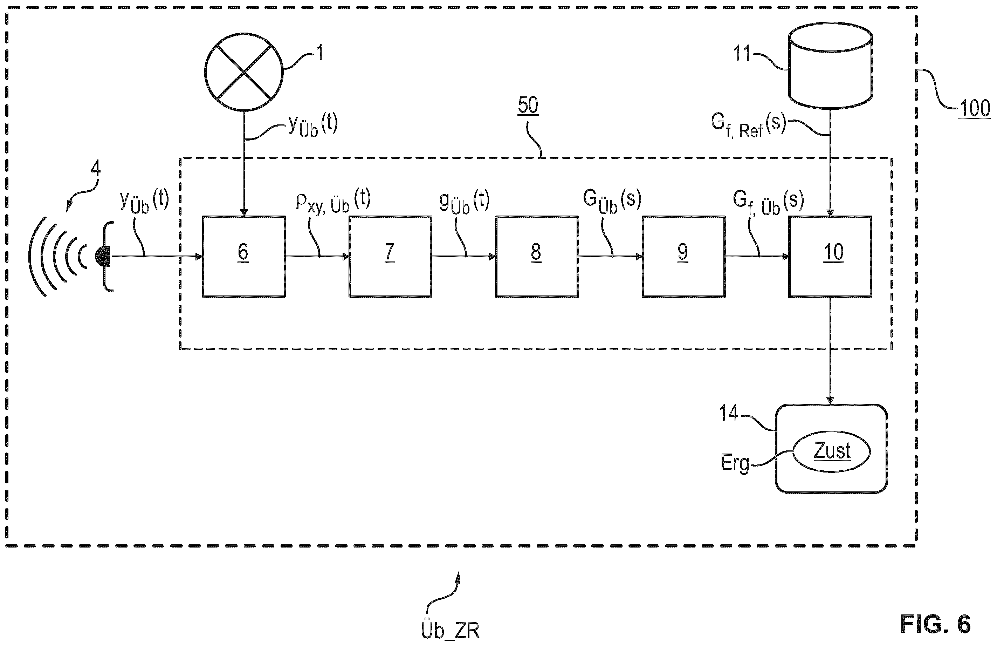

Nachfolgend wird diese Signalverarbeitungseinheit 50 näher beschrieben, und zwar mit Bezug auf Figur 4 und Figur 6.

Im Ausführungsbeispiel umfasst die Signalverarbeitungseinheit 50 mindestens einen Prozessor, einen Datenspeicher 11 sowie folgende funktionale Bestandteile, die bevorzugt als Softwareprogramme ausgestaltet sind, die auf dem Prozessor ablaufen, während das Gasdetektionsgerät 100 eingesetzt wird:
- einen Kreuzkorrelator 6,
- einen optionalen Extrahierer 7,
- einen Transformierer 8,
- eine optionale Filtereinheit 9 und
- eine Vergleichseinheit 10.

Der Kreuzkorrelator 6 berechnet ein Maß für die Kreuzkorrelation ρ_{xy,Ref} = ρ_{xy,Ref}(τ) und ρ_{xy,Üb} = ρ_{xy,Üb}(τ) zwischen dem Eingangssignal x_{Ref}(t), x_{Üb}(t) und dem Ausgangssignal y_{Ref}(t), y_{Üb}(t). Die Kreuzkorrelation ρ_{xy}(τ) beschreibt die Ähnlichkeit zwischen zwei Signalen x(t) und y(t). Bekanntlich wird die Kreuzkorrelation ρ_{xy}(τ) im Zeitbereich gemäß der Formel ${ρ}_{xy} \left(τ\right) = \begin{matrix}lim \\ T \to ∞\end{matrix} \frac{1}{T} {\int }_{- T / 2}^{+ T / 2} x \left(t\right) y \left(t+τ\right) dt$ (1) berechnet. Das Ausgangssignal y(t) eines linearen zeitinvarianten Systems hängt bekanntlich mit dem Eingangssignal x(t) durch die sogenannte Impulsantwort (impulse response) g(t) zusammen, nämlich gemäß der Formel $y \left(t\right) = g \left(t\right) * x \left(t\right) = {\int }_{- ∞}^{+ ∞} g \left(u\right) x \left(t-u\right) du$ (2)

Hierbei ist g(t) * x(t) die Faltung (convolution) zwischen den beiden Signalen g und x.

Die Kreuzkorrelation ρ_{xy}(τ) hängt bekanntlich mit der Impulsantwort g(t) gemäß der Formel ${ρ}_{xy} \left(τ\right) = {\int }_{- ∞}^{+ ∞} g \left(u\right) {ρ}_{xx} \left(τ-u\right) du$ (3) zusammen. Hierbei ist ρₓₓ die Autokorrelation des Eingangssignals x(t).

Nachfolgend bezeichnet der Index Ref eine Größe, die mit Hilfe des Ausgangssignals y_{Ref}(t) aus dem Referenz-Zeitraum Ref_ZR generiert wurde, der Index Üb eine Größe, die mithilfe des Ausgangssignals y_{Üb}(t) aus dem Überprüfungs-Zeitraum Üb_ZR generiert wurde.

Auf Basis der gerade dargelegten Zusammenhänge generiert der Extrahierer 7 aus der Kreuzkorrelation ρ_{xy,Ref}(τ) die Impulsantwort g_{Ref}(t) und aus der Kreuzkorrelation ρ_{xy,Üb}(τ) die Impulsantwort g_{Üb}(t).

Der Transformierer 8 transformiert die Impulsantworten g_{Ref}(t) und g_{Üb}(t) vom Zeitbereich in den Frequenzbereich, und zwar im Ausführungsbeispiel durch eine Laplace-Transformation. Durch diese Laplace-Transformation werden die Übertragungsfunktion G_{Ref}(s) der Impulsantwort g_{Ref}(t) und die Übertragungsfunktion G_{Üb}(S) der Impulsantwort g_{Üb}(t) berechnet.

Bekanntlich wird die Übertragungsfunktion G(s) im Komplexen einer Impulsantwort g(t) durch eine Laplace-Transformation gemäß der folgenden Rechenvorschrift berechnet: $G \left(s\right) = L \left\{g\left(t\right)\right\} = {\int }_{- ∞}^{+ ∞} g \left(t\right) exp \left(-s t\right) dt$ (4) mit s = σ+jω, wobei σ der Realteil, j die imaginäre Zahl und ω = 2πf die Kreisfrequenz sind.

In einer alternativen Realisierung wird der folgende Zusammenhang ausgenutzt, um die Übertragungsfunktionen G_{Ref}(S) und G_{Üb}(S) zu berechnen: ${G}_{Ref} \left(s\right) = \frac{L \left\{{y}_{Ref}\left(t\right)\right\}}{L \left\{{x}_{Ref}\left(t\right)\right\}} und {G}_{Üb} \left(s\right) = \frac{L \left\{{y}_{Üb}\left(t\right)\right\}}{L \left\{{x}_{Üb}\left(t\right)\right\}}$ (5)

Bei dieser Ausgestaltung werden also die Laplace-Transformierte der Eingangssignale x_{Ref}(t), x_{Üb}(t) und die Laplace-Transformierte der Ausgangssignale y_{Ref}(t), y_{Üb}(t)berechnet. Die Ausgestaltung vermeidet die Notwendigkeit, eine Kreuzkorrelation zu berechnen.

Die optionale Filtereinheit 9 erzeugt aus der Übertragungsfunktion G_{Ref}(s) die gefilterte Übertragungsfunktion G_{f,Ref}(S) und aus der Übertragungsfunktion G_{Üb}(S) die gefilterte Übertragungsfunktion G_{f,Üb}(S).

Die Übertragungsfunktion G_{Ref}(S) oder die gefilterte Übertragungsfunktion G_{f,Ref}(S) aus dem Referenz-Zeitraum Ref_ZR wird im Datenspeicher 11 abgespeichert und für die Überprüfung wieder aus dem Datenspeicher 11 ausgelesen.

Die Vergleichseinheit 10 vergleicht die Übertragungsfunktion G_{Üb}(S) aus dem Überprüfungs-Zeitraum Üb_ZR mit der Übertragungsfunktion G_{Ref}(S) aus dem Referenz-Zeitraum Ref_ZR, optional die beiden gefilterten Übertragungsfunktionen G_{f,Üb}(S) und G_{f,Ref}(S) miteinander. Idealerweise stimmen diese beiden Übertragungsfunktionen bei einem intakten Gasdetektionsgerät 100 miteinander überein. Eine Abweichung oberhalb einer vorgegebenen Toleranz ist ein Hinweis auf einen Fehler. Bevorzugt ermittelt die Vergleichseinheit 10 den Phasengang und den Amplitudengang. In vielen Fällen liefert der Vergleich der Phasengänge einen Hinweis, in welchen Frequenzen und damit bei welchen Zielgasen ein Fehler auftritt. Der Vergleich der Amplitudengänge kann ein Hinweis darauf sein, bei welchen Zielgas-Konzentrationen ein Fehler auftritt.

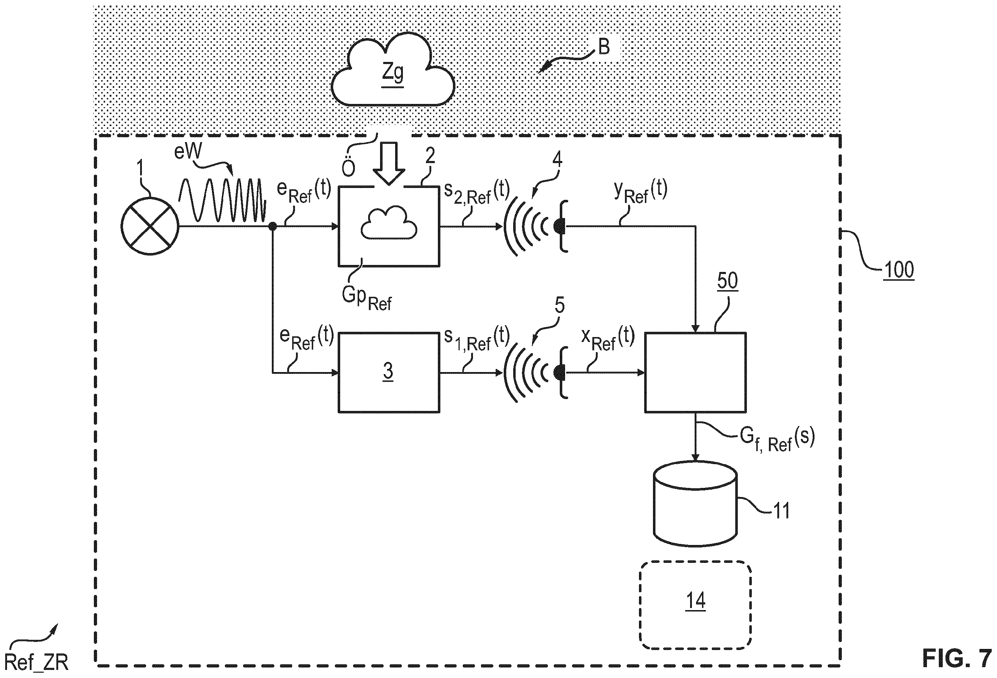

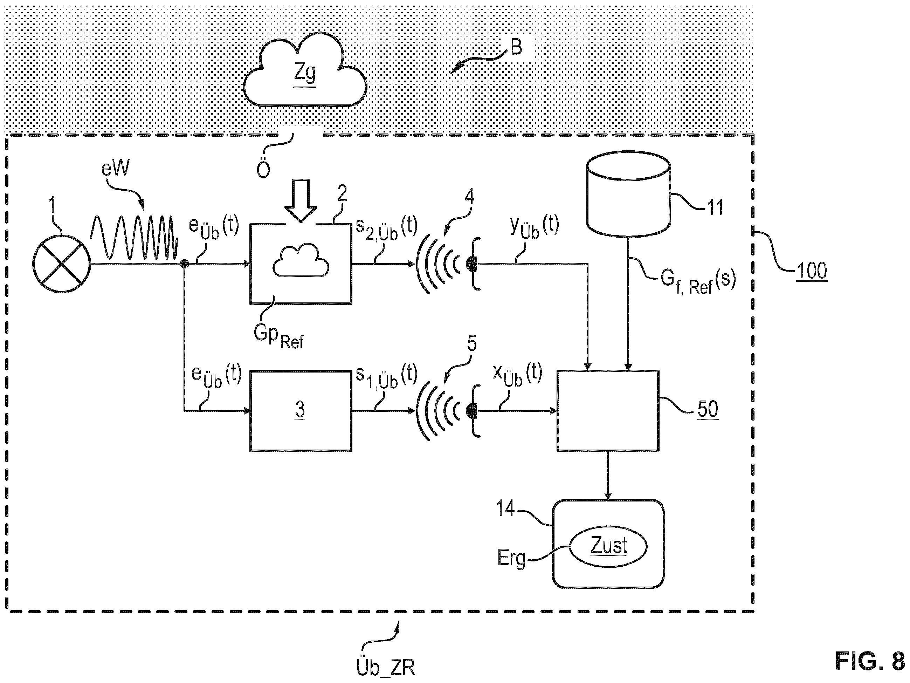

Figur 7 und Figur 8 zeigen eine alternative Ausgestaltung, wobei sich Figur 7 wiederum auf den Referenz-Zeitraum Ref_ZR bezieht und Figur 8 auf den Überprüfungs-Zeitraum Üb_ZR. Gleiche Bezugszeichen haben die gleichen Bedeutungen wie in Figur 3 bis Figur 6.

Gemäß dieser Ausgestaltung umfasst das Gasdetektionsgerät 100 zusätzlich zur Messkammer 2 eine Referenzkammer 3. Die Referenzkammer 3 ist mit einem Referenzgas Rg gefüllt, welches frei von dem oder jedem zu detektierenden Zielgas ist. Die Strahlungsquelle 1 emittiert Strahlung eW sowohl in die Messkammer 2 als auch in die Referenzkammer 3, und beide Kammern 2, 3 sind parallel zur Strahlungsquelle 1 angeordnet. Das Signal, welches die zeitlich veränderliche Intensität der emittierten Strahlung eW beschreibt, bevor diese Strahlung die Kammern 2, 3 erreicht, wird mit e_{Ref}(t) bzw. e_{Üb}(t) bezeichnet. Die Intensität der Strahlung eW, welche mindestens einmal die Messkammer 2 und daher eine Gasprobe Gp_{Ref}, Gp_{Üb} in der Messkammer 2 durchdrungen hat, wird mit s_{2,Ref}(t) bzw. s_{2,Üb}(t) bezeichnet. Die Intensität der Strahlung eW, welche mindestens einmal die Referenzkammer 3 durchdrungen hat, wird mit s_{1,Ref}(t) bzw. s_{1,Üb}(t) bezeichnet.

Genauso wie bei der Ausgestaltung gemäß Figur 3 bis Figur 6 erzeugt der Detektor 4 auch bei der Ausgestaltung gemäß Figur 7 und Figur 8 ein Ausgangssignal y_{Ref}(t), y_{Üb}(t), das von der Intensität s_{2,Ref}(t) bzw. S_{2,Üb}(t) der auftreffenden Strahlung eW abhängt. Ein Referenz-Detektor 5 erzeugt abhängig von auftreffender Strahlung eW ein Signal. Dieses Signal hängt von der Intensität s_{1,Ref}(t) bzw. s_{1,Üb}(t) der Strahlung eW, die mindestens einmal die Referenzkammer 3 durchdrungen hat und auf den Referenz-Detektor 5 auftrifft, ab. Abweichend wird dieses Signal, das der Referenz-Detektor 5 generiert hat, als das Eingangssignal x_{Ref}(t), x_{Üb}(t) verwendet. Das gedachte System, dessen Systemverhalten berechnet wird, wird also mit dem Signal x_{Ref}(t), x_{Üb}(t) des Referenz-Detektors 5 angeregt und erzeugt als Reaktion auf diese Anregung das Ausgangssignal y_{Ref}(t), y_{Üb}(t). Die weiteren Schritte bei der Überprüfung sind die gleichen, die weiter oben mit Bezug auf Figur 3 bis Figur 6 beschrieben wurden.

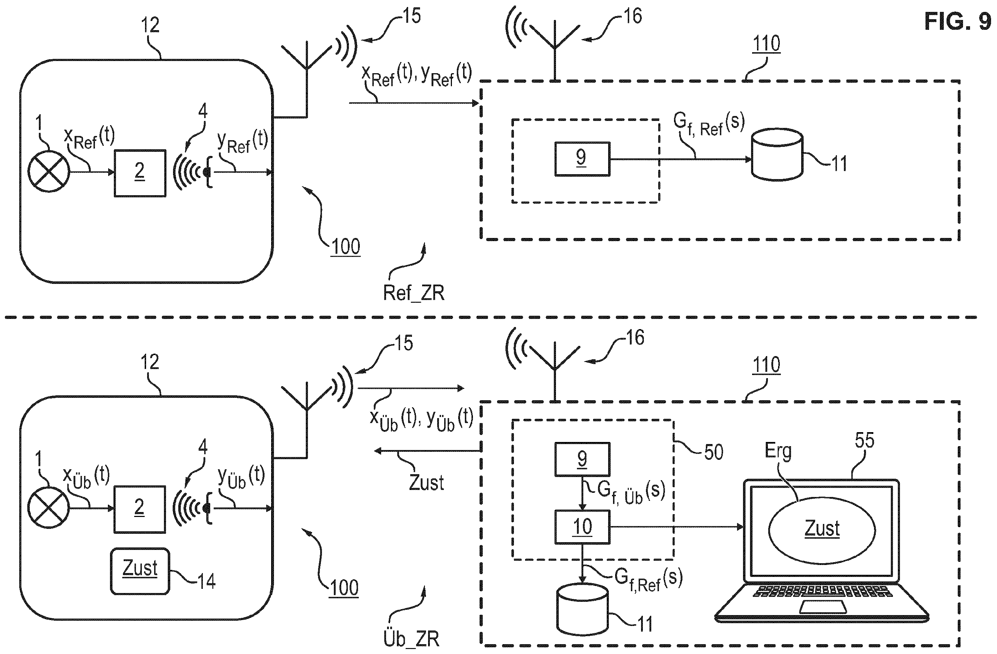

In der Ausgestaltung gemäß Figur 3 bis Figur 8 ist die Signalverarbeitungseinheit 50 ein Bestandteil des Gasdetektionsgeräts 100. Figur 9 zeigt eine alternative Ausgestaltung.

Die Signalverarbeitungseinheit 50 ist gemäß dieser alternativen Ausgestaltung ein Bestandteil einer Rechnereinheit 110, die räumlich vom Gasdetektionsgerät 100 entfernt ist und mit dieser wenigstens zeitweise in einer Datenverbindung steht. Die Datenverbindung kann durch Kabel oder durch Funkwellen hergestellt sein. Diese Ausgestaltung erleichtert es, die Signalverarbeitungseinheit 50 auf einem leistungsfähigen Rechner zu realisieren. Das Gasdetektionsgerät 100 umfasst in der gezeigten Realisierungsform eine Kommunikationseinheit 15, die per Funkwellen Nachrichten mit einer Kommunikationseinheit 16 der Rechnereinheit 110 austauscht. Das Gasdetektionsgerät 100 übermittelt im Referenz-Zeitraum Ref_ZR das Eingangssignal x_{Ref}(t) und das Ausgangssignal y_{Ref}(t) sowie im Überprüfungs-Zeitraum Üb_ZR das Eingangssignal x_{Üb}(t) und das Ausgangssignal y_{Üb}(t) an die Rechnereinheit 110. Die Vergleichseinheit 10 erzeugt ein Ergebnis Erg mit einer Zustands-Information Zust. Im gezeigten Beispiel wird diese Zustands-Information Zust einerseits auf einem Ausgaberechner 55 der Rechnereinheit 110 angezeigt. Andererseits wird die Zustands-Information Zust zurück an das Gasdetektionsgerät 100 übermittelt und dort auf der Ausgabeeinheit 14 ausgegeben.

### Bezugszeichenliste

| | |
|---|---|
| 1 | Strahlungsquelle, emittiert in die Messkammer 2 elektromagnetische oder akustische Strahlung eW, deren Intensität durch das Eingangssignal x_{Ref}(t), x_{Üb}(t) bzw. e_{Ref}(t), e_{Üb}(t) beschrieben wird |
| 2 | Messkammer, nimmt die Gasprobe Gp_{Ref}, Gp_{Üb} auf |
| 3 | Referenzkammer, nimmt das Referenzgas Rg auf |
| 4 | Detektor an der Messkammer 2, liefert im Referenz-Zeitraum Ref_ZR das zeitaufgelöste Ausgangssignal y_{Ref}(t) und im Überprüfungs-Zeitraum Üb_ZR das zeitaufgelöste Ausgangssignal Y_{Üb}(t) |
| 5 | Referenz-Detektor an der Referenzkammer 3, liefert im Referenz-Zeitraum Ref_ZR das zeitaufgelöste Eingangssignal x_{Ref}(t) und im Überprüfungs-Zeitraum Üb_ZR das zeitaufgelöste Eingangssignal x_{Üb}(t) |
| 6 | Kreuzkorrelator, berechnet im Referenz-Zeitraum Ref_ZR die Kreuzkorrelation ρ_{xy,Ref}(t) zwischen dem Eingangssignal x_{Ref}(t) und dem Ausgangssignal y_{Ref}(t) und im Überprüfungs-Zeitraum Üb_ZR die Kreuzkorrelation ρ_{xy,Üb}(t) zwischen dem Eingangssignal x_{Üb}(t) und dem Ausgangssignal y_{Üb}(t) |
| 7 | Extrahierer, erzeugt im Referenz-Zeitraum Ref_ZR aus der Kreuzkorrelation ρ_{xy,Ref}(t) die Impulsantwort g_{Ref}(t) und im Überprüfungs-Zeitraum Üb_ZR aus der Kreuzkorrelation ρ_{xy,Üb}(t) die Impulsantwort g_{Üb}(t) |
| 8 | Transformierer, transformiert die Impulsantwort g_{Ref}(t), g_{Üb}(t) aus dem Zeitbereich in den Frequenzbereich, liefert das Transformationsergebnis G_{Ref}(S), G_{Üb}(S) |
| 9 | Filtereinheit, erzeugt aus der Übertragungsfunktion G_{Ref}(s) die gefilterte Übertragungsfunktion G_{f,Ref}(S) und aus der Übertragungsfunktion G_{Üb}(S) die gefilterte Übertragungsfunktion G_{f,Üb}(S) |
| 10 | Vergleichseinheit, vergleicht die beiden Maße G_{f,Ref}(S), G_{f,Üb}(S) für das Systemverhalten miteinander, liefert als Ergebnis eine Information Zust über den aktuellen Zustand des Gasdetektionsgeräts 100 |
| 11 | Datenspeicher, in dem im Referenz-Zeitraum Ref_ZR das Maß G_{f,Ref}(S) für das Referenz-Systemverhalten abgespeichert wird |
| 12 | Gehäuse des Gasdetektionsgeräts 100 |
| 14 | Ausgabeeinheit des Gasdetektionsgeräts 100 oder der Rechnereinheit 110, gibt die Zustands-Information Zust des Gasdetektionsgeräts 100 visuell und / oder akustisch und / oder haptisch aus |
| 15 | Kommunikationseinheit des Gasdetektionsgeräts 100 |
| 16 | Kommunikationseinheit der Rechnereinheit 110 |
| 50 | Signalverarbeitungseinheit, umfasst den Kreuzkorrelator 6, den Extrahierer 7, den Transformierer 8, die optionale Filtereinheit 9 und die Vergleichseinheit 10, hat Lesezugriff auf den Datenspeicher 11, in einer Ausgestaltung Bestandteil des Gasdetektionsgeräts 100 und in einer anderen Ausgestaltung ein Bestandteil der Rechnereinheit 110 |
| 55 | Ausgaberechner mit Bildschirm, zeigt die Zustands-Information Zust an |
| 100 | zu überprüfendes Gasdetektionsgerät, umfasst die Messkammer 2, den Detektor 4, die Strahlungsquelle 1, das Gehäuse 12, den Datenspeicher 11 sowie in einer Ausgestaltung die Signalverarbeitungseinheit 50 und in einer anderen Ausgestaltung die Kommunikationseinheit 15 |
| 110 | räumlich entfernte Rechnereinheit, umfasst die Signalverarbeitungseinheit 50, den Ausgaberechner 55 und die Kommunikationseinheit 16, mit dem Gasdetektionsgerät 100 drahtlos verbunden |
| B | zu überwachender räumlicher Bereich, kann mindestens ein Zielgas Zg aufweisen |
| Erg | Ergebnis der Vergleichseinheit 10, umfasst die Zustands-Information Zust |
| e_{Ref}(t) | Intensität der Strahlung eW vor dem Durchdringen der beiden Kammern 2, 3 im Referenz-Zeitraum Ref_ZR |
| e_{Üb}(t) | Intensität der Strahlung eW vor dem Durchdringen der beiden Kammern 2, 3 im Überprüfungs-Zeitraum Üb_ZR |
| eW | elektromagnetische oder akustische Strahlung, von der Strahlungsquelle 1 in die Messkammer 2 emittiert, weist vor dem Durchgang durch die Messkammer 2 die Intensität x_{Ref}(t), x_{Üb}(t) und nach dem Durchgang die Intensität s_{Ref}(t), s_{Üb}(t) auf |
| Gp_{Ref} | Gasprobe, fließt vor dem oder im Referenz-Zeitraum Ref_ZR aus dem zu überwachenden Bereich B in die Messkammer 2 |
| Gp_{Üb} | Gasprobe, fließt vor dem oder im Überprüfungs-Zeitraum Üb_ZR aus dem zu überwachenden Bereich B in die Messkammer 2 |
| g_{Ref}(t) | Impulsantwort im Zeitbereich, wird im Referenz-Zeitraum Ref_ZR vom Extrahierer 7 aus der Kreuzkorrelation ρ_{xy,Ref}(t) erzeugt |
| g_{Üb}(t) | Impulsantwort im Zeitbereich, wird im Überprüfungs-Zeitraum Üb_ZR vom Extrahierer 7 aus der Kreuzkorrelation ρ_{xy,Üb}(t) erzeugt |
| G_{Ref}(s) | Übertragungsfunktion des Systems, das als Reaktion auf eine Anregung durch das Eingangssignal x_{Ref}(t) das Ausgangssignal y_{Ref}(t) liefert, beschreibt das Systemverhalten im Referenz-Zeitraum Ref_ZR, wird vom Transformierer 8 geliefert |
| G_{Üb}(s) | Übertragungsfunktion des Systems, das als Reaktion auf eine Anregung durch das Eingangssignal x_{Üb}(t) das Ausgangssignal y_{Üb}(t)liefert, beschreibt das Systemverhalten im Überprüfungs-Zeitraum Üb_ZR, wird vom Transformierer 8 geliefert |
| G_{f,Ref}(s) | gefilterte Übertragungsfunktion im Frequenzbereich, von der Filtereinheit 9 im Referenz-Zeitraum Ref_ZR geliefert |
| G_{f,Üb}(s) | gefilterte Übertragungsfunktion im Frequenzbereich, von der Filtereinheit 9 im Überprüfungs-Zeitraum Üb_ZR geliefert |
| Ö | Öffnung in der Messkammer 2 |
| ρₓₓ(t) | Autokorrelation des Eingangssignals x(t) |
| ρ_{xy,Ref}(t) | Kreuzkorrelation im Zeitbereich zwischen dem Eingangssignal x_{Ref}(t) und dem Ausgangssignal y_{Ref}(t) im Referenz-Zeitraum Ref_ZR, vom Kreuzkorrelator 6 berechnet |
| ρ_{xy,Üb}(t) | Kreuzkorrelation im Zeitbereich zwischen dem Eingangssignal x_{Üb}(t) und dem Ausgangssignal y_{Üb}(t)im Überprüfungs-Zeitraum Üb_ZR, vom Kreuzkorrelator 6 berechnet |
| Ref_ZR | Referenz-Zeitraum, in dem das Gasdetektionsgerät 100 intakt ist |
| Rg | Referenzgas in der Referenzkammer 3, frei von jedem Zielgas |
| s_{Ref}(t) | Signal, das die Intensität der Strahlung eW nach dem Durchdringen der Messkammer 2 im Referenz-Zeitraum Ref_ZR beschreibt |
| s_{Üb}(t) | Signal, das die Intensität der Strahlung eW nach dem Durchdringen der Messkammer 2 im Überprüfungs-Zeitraum Üb_ZR beschreibt |
| s_{1,Ref}(t) | Signal, das die Intensität der Strahlung eW nach dem Durchdringen der Referenzkammer 3 im Referenz-Zeitraum Ref_ZR beschreibt |
| s_{1,Üb}(t) | Signal, das die Intensität der Strahlung eW nach dem Durchdringen der Referenzkammer 3 im Überprüfungs-Zeitraum Üb_ZR beschreibt |
| Üb_ZR | Überprüfungs-Zeitraum, in dem das Gasdetektionsgerät 100 fehlerhaft sein kann |
| x_{Ref}(t) | zeitaufgelöstes Eingangssignal, beschreibt die Intensität der Strahlung eW, die die Strahlungsquelle 1 im Referenz-Zeitraum Ref_ZR in die Messkammer 2 emittiert |
| x_{Üb}(t) | zeitaufgelöstes Eingangssignal, beschreibt die Intensität der Strahlung eW, die die Strahlungsquelle 1 im Überprüfungs-Zeitraum Üb_ZR in die Messkammer 2 emittiert |
| y_{Ref}(t) | zeitaufgelöstes Ausgangssignal, vom Detektor 4 als Reaktion auf das Eingangssignal x_{Ref}(t) im Referenz-Zeitraum Ref_ZR geliefert |
| y_{Üb}(t) | zeitaufgelöstes Ausgangssignal, vom Detektor 4 als Reaktion auf das Eingangssignal x_{Üb}(t) im Überprüfungs-Zeitraum Üb_ZR geliefert |
| Zg | zu detektierendes Zielgas |
| Zust | Zustands-Information über den aktuellen Zustand des Gasdetektionsgeräts 100 |

## Patentansprüche

1. Überprüfungs-Verfahren zum Überprüfen eines Gasdetektionsgeräts (100),
wobei das zu überprüfende Gasdetektionsgerät (100)
- eine Messkammer (2),
- einen Detektor (4) und
- eine Strahlungsquelle (1)
umfasst,
wobei die Messkammer (2) dazu ausgestaltet ist, eine zu untersuchende Gasprobe (Gp_{Üb}, Gp_{Ref}) aufzunehmen,
wobei das Überprüfungs-Verfahren die automatisch durchgeführten Schritte umfasst, dass
sowohl in einem Referenz-Zeitraum (Ref_ZR) als auch in einem vom Referenz-Zeitraum (Ref_ZR) verschiedenen Überprüfungs-Zeitraum (Üb_ZR) jeweils
- die Strahlungsquelle (1) Strahlung (eW) in die Messkammer (2) emittiert, insbesondere elektromagnetische Strahlung,
wobei als ein jeweiliges Eingangssignal [x_{Ref}(t), x_{Üb}(t)] ein Maß für die Intensität der emittierten Strahlung (eW) vor dem Eintritt in die Messkammer (2) vorgegeben oder gemessen wird,
- wenigstens ein Teil der emittierten Strahlung (eW) mindestens einmal wenigstens einen Teil der Gasprobe (Gp_{Üb}, Gp_{Ref}) in der Messkammer (2) durchdringt, so dass die Strahlung (eW) in der Messkammer (2) eine vom Eingangssignal [x_{Ref}(t), x_{Üb}(t)] abhängende Intensität [s_{Ref}(t), s_{Üb}(t)] aufweist,
- der Detektor (4) abhängig von der Intensität [S_{Ref}(t), s_{Üb}(t)] der Strahlung (eW) in der Messkammer (2) ein Ausgangssignal [y_{Ref}(t), y_{Üb}(t)] generiert, wobei die gemessene Intensität [s_{Ref}(t), s_{Üb}(t)] auftritt, nachdem die Strahlung (eW) wenigstens einen Teil der Gasprobe (Gp_{Üb}, Gp_{Ref}) in der Messkammer (2) mindestens einmal durchdrungen hat, und
- im Zeitbereich oder im Frequenzbereich ein Maß [G_{Ref}(S), G_{Üb}(S)] für ein Systemverhalten eines gedachten Systems berechnet wird,
wobei dieses gedachte System mit dem Eingangssignal [x_{Ref}(t), x_{Üb}(t)] angeregt wird und dieses System als Reaktion auf diese Anregung das Ausgangssignal [y_{Ref}(t), Y_{Üb}(t)] erzeugt,
wobei die Strahlungsquelle (1), die Messkammer (2) und der Detektor (4) im Referenz-Zeitraum (Ref_ZR) intakt sind und
wobei das Verfahren die weiteren automatisch durchgeführten Schritte umfasst, dass
- das Überprüfungs-Systemverhalten, das ist das Systemverhalten des Systems, das im Überprüfungs-Zeitraum (Üb_ZR) mit dem Eingangssignal [x_{Üb}(t)] angeregt wird und als Reaktion das Ausgangssignal [y_{Üb} (t)] erzeugt, mit
- dem Referenz-Systemverhalten, das ist das Systemverhalten des Systems, das im Referenz-Zeitraum (Ref_ZR) mit dem Eingangssignal [x_{Ref}(t)] angeregt wird und als Reaktion das Ausgangssignal [y_{Ref}(t)] erzeugt,
verglichen wird und
wobei für den Vergleich das im Überprüfungs-Zeitraum (Üb_ZR) berechnete Maß [G_{Üb}(S)] für das Systemverhalten und das im Referenz-Zeitraum (Ref_ZR) berechneten Maß [G_{Ref}(S)] für das Systemverhalten verwendet werden, und
abhängig vom Ergebnis des Vergleichs zwischen den beiden Systemverhalten eine Zustands-Information (Zust) über den Zustand des Gasdetektionsgeräts (100) im Überprüfungs-Zeitraum (Üb_ZR) hergeleitet wird.

2. Überprüfungs-Verfahren nach Anspruch 1,
**dadurch gekennzeichnet, dass**
sowohl im Referenz-Zeitraum (Ref_ZR) als auch im Überprüfungs-Zeitraum (Üb_ZR) der Schritt, das Maß [G_{Ref}(S), G_{Üb}(S)] für das Systemverhalten zu berechnen, jeweils den Schritt umfasst, dass
ein Maß für die Kreuzkorrelation [ρ_{xy,Ref}(t), ρ_{xy,Üb}(t)] zwischen dem Eingangssignal [x_{Ref}(t), x_{Üb}(t)] und dem Ausgangssignal [y_{Ref}(t), Y_{Üb}(t)] berechnet wird.

3. Überprüfungs-Verfahren nach Anspruch 2,
**dadurch gekennzeichnet, dass**
sowohl im Referenz-Zeitraum (Ref_ZR) als auch im Überprüfungs-Zeitraum (Üb_ZR) der Schritt, das Maß [G_{Ref}(S), G_{Üb}(S)] für das Systemverhalten zu berechnen, jeweils die zusätzlichen Schritte umfasst, dass
- eine Transformation der Kreuzkorrelation [ρ_{xy,Ref}(t), ρ_{xy,Üb}(t)] in den Frequenzbereich durchgeführt wird,
insbesondere durch eine Laplace-Transformation oder eine Fourier-Transformation, und
- das Maß [G_{Ref}(S), G_{Üb}(S)] für das Systemverhalten unter Verwendung des Ergebnisses der Transformation der Kreuzkorrelation [ρ_{xy,Ref}(t), ρ_{xy,Üb}(t)] in den Frequenzbereich ermittelt wird.

4. Überprüfungs-Verfahren nach Anspruch 3,
**dadurch gekennzeichnet, dass**
in beiden Zeiträumen (Ref_ZR, Üb_ZR) die Schritte durchgeführt werden, dass
- die Strahlungsquelle (1) in beiden Zeiträumen (Ref_ZR, Üb_ZR) jeweils die Strahlung (eW) mit einem Eingangssignal [x_{Ref}(t), x_{Üb}(t)] in Form eines weißen Rauschens in die Messkammer (2) emittiert,
- unter Verwendung der Transformation der Kreuzkorrelation [ρ_{xy,Ref}(t), ρ_{xy,Üb}(t)] in den Frequenzbereich die jeweilige Übertragungsfunktion [G_{Ref}(S), G_{Üb}(S)] des gedachten Systems bei einer Anregung durch das Eingangssignal [x_{Ref}(t), x_{Üb}(t)] berechnet wird und
- das jeweilige Maß [G_{Ref}(S), G_{Üb}(S)] für das Systemverhalten unter Verwendung der jeweiligen Übertragungsfunktion berechnet wird.

5. Überprüfungs-Verfahren nach einem der vorhergehenden Ansprüche,
**dadurch gekennzeichnet, dass**
die Intensität der emittierten Strahlung (eW) in einem Frequenzband größer oder gleich einem unteren Schwellwert ist und
die Messkammer (2) im Referenz-Zeitraum (Ref_ZR) mit einer Referenz-Gasprobe (Gp_{Ref}) und im Überprüfungs-Zeitraum (Üb_ZR) mit einer Überprüfungs-Gasprobe (Gp_{Üb}) gefüllt wird oder ist,
wobei die beiden Gasproben (Gp_{Ref}, Gp_{Üb}) im Frequenzband die Strahlung (eW) bis auf eine vorgegebene Toleranz gleich stark abschwächen und
wobei insbesondere die jeweilige chemische Zusammensetzung der beiden Gasproben (Gp_{Ref}, Gp_{Üb}) sich um höchstens eine vorgegebene Zusammensetzungs-Toleranz voneinander unterscheiden.

6. Überprüfungs-Verfahren nach einem der vorhergehenden Ansprüche,
**dadurch gekennzeichnet, dass**
eine Zielgas-Menge mit mindestens einem zu detektierenden Zielgas (Zg) vorgegeben ist oder wird, bevorzugt mit mehreren Zielgasen, und
das Frequenzband der Strahlung (eW), die die Strahlungsquelle (1) emittiert, für das oder jedes Zielgas der Zielgas-Menge jeweils ein Zielgas-Frequenzband überdeckt,
wobei das Zielgas (Zg) Strahlung (eW) im jeweiligen Zielgas-Frequenzband stärker als eine vorgegebene untere Schranke absorbiert.

7. Überprüfungs-Verfahren nach einem der vorhergehenden Ansprüche,
**dadurch gekennzeichnet, dass**
das Überprüfungs-Verfahren unter Verwendung einer Signalverarbeitungseinheit (50) durchgeführt wird,
wobei die Signalverarbeitungseinheit (50) räumlich vom Gasdetektionsgerät (100) beabstandet ist und
wobei das Überprüfungs-Verfahren die zusätzlichen Schritte umfasst, dass in beiden Zeiträumen (Ref_ZR, Üb_ZR) das jeweilige Eingangssignal [x_{Ref}(t), x_{Üb}(t)] und das jeweilige Ausgangssignal [y_{Ref}(t), y_{Üb}(t)] vom Gasdetektionsgerät (100) an die Signalverarbeitungseinheit (50) übermittelt werden und
die Signalverarbeitungseinheit (50) die Schritte durchführt,
- die Maße [G_{Ref}(S), G_{Üb}(S)] für das Überprüfungs-Systemverhalten und das Referenz-Systemverhalten zu berechnen,
- diese miteinander zu vergleichen und
- die Zustands-Information (Zust) herzuleiten und
- zu bewirken, dass die Zustands-Information (Zust) in mindestens einer von einem Menschen wahrnehmbaren Form ausgegeben wird.

8. Überprüfungs-Verfahren nach einem der vorhergehenden Ansprüche,
**dadurch gekennzeichnet, dass**
das Gasdetektionsgerät (100) zusätzlich eine Referenzkammer (3) und einen Referenz-Detektor (5) umfasst,
wobei die Referenzkammer (3) mit einem Referenzgas (Rg) gefüllt ist, das frei von dem oder jedem Zielgas ist,
wobei die Strahlungsquelle (1) sowohl im Referenz-Zeitraum (Ref_ZR) als auch im Überprüfungs-Zeitraum (Üb_ZR) Strahlung [eW, e_{Ref}(t), e_{Üb}(t)] sowohl in die Messkammer (2) als auch in die Referenzkammer (3) emittiert,
so dass emittierte Strahlung [eW, e_{Ref}(t), e_{Üb}(t)] beide Kammern (2, 3) jeweils mindestens einmal durchdringt,
wobei die beiden Kammern (2, 3) bezüglich der Strahlung [eW, e_{Ref}(t), e_{Üb}(t)] parallel angeordnet sind,
wobei der Referenz-Detektor (5) sowohl im Referenz-Zeitraum (Ref_ZR) als auch im Überprüfungs-Zeitraum (Üb_ZR) jeweils abhängig von der Intensität von emittierter Strahlung (eW) in der Referenzkammer (3) ein zeitaufgelöstes Signal [x_{Ref}(t), x_{Üb}(t)] erzeugt und
wobei als das gedachte System ein System verwendet wird, das mit dem Signal vom Referenz-Detektor (5) als dem Eingangssignal [x_{Ref}(t), x_{Üb}(t)] angeregt wird und als Reaktion auf diese Anregung das Ausgangssignal [y_{Ref}(t), y_{Üb}(t)] erzeugt.

9. Überprüfungs-Verfahren nach einem der vorhergehenden Ansprüche,
**dadurch gekennzeichnet, dass**
sowohl im Referenz-Zeitraum (Ref_ZR) als auch im Überprüfungs-Zeitraum (Üb_ZR) jeweils der Detektor (4) das Ausgangssignal [y_{Ref}(t), y_{Üb}(t)] abhängig von der Intensität [S_{Ref}(t), s_{Üb}(t)] der Strahlung (eW) in der Messkammer (2) und von dem Wert eines Parameters erzeugt,
wobei mehrere mögliche Werte für den Parameter vorgegeben sind und
wobei das Verfahren die weiteren automatisch durchgeführten Schritte umfasst, dass
im Überprüfungs-Zeitraum (Üb_ZR)
- für jeden möglichen Wert des Parameters das bei diesem möglichen Parameter-Wert resultierende Maß [G_{Üb}(S)] für das Überprüfungs-Systemverhalten berechnet und mit dem Maß [G_{Ref}(S)] für das Referenz-Systemverhalten verglichen wird und
- ein Maß für die Abweichung zwischen dem bei diesem möglichen Parameter-Wert resultierende Überprüfungs-Systemverhalten und dem Referenz-Systemverhalten [G_{Ref}(S)] ermittelt wird und
abhängig von den Abweichungs-Maßen für die möglichen Parameter-Werte ein Wert für den Parameter festgelegt und verwendet wird,
insbesondere derjenige Parameter-Wert verwendet wird, der zum geringsten Abweichungs-Maß führt.

10. Mess-Verfahren zur Messung der Konzentration eines Zielgases (Zg)
unter Verwendung eines Gasdetektionsgeräts (100), welches
- eine Messkammer (2),
- einen Detektor (4) und
- eine Strahlungsquelle (1)
umfasst,
wobei das Mess-Verfahren die automatisch durchgeführten Schritte umfasst, dass
- die Messkammer (2) eine zu untersuchende Gasprobe (Gp_{Üb}) aufnimmt,
- die Strahlungsquelle (1) Strahlung (eW) in die Messkammer (2) emittiert,
- wenigstens ein Teil der emittierten Strahlung (eW) mindestens einmal wenigstens einen Teil der Gasprobe (Gp_{Üb}) in der Messkammer (2) durchdringt, so dass die Strahlung (eW) in der Messkammer (2) eine vom Eingangssignal [x_{Üb}(t)] und von der Gasprobe (Gp_{Üb}) abhängende Intensität aufweist,
- der Detektor (4) abhängig von der Intensität der Strahlung (eW) in der Messkammer (2) ein Ausgangssignal [y_{Üb}(t)] generiert,
wobei die gemessene Intensität auftritt, nachdem die Strahlung (eW) wenigstens einen Teil der Gasprobe in der Messkammer (2) mindestens einmal durchdrungen hat, und
- die Zielgas-Konzentration abhängig vom Ausgangssignal [y_{Üb}(t)] gemessen wird, und
wobei mindestens einmal für das Gasdetektionsgerät (100) ein Überprüfungs-Verfahren nach einem der vorhergehenden Ansprüche durchgeführt wird.

11. Überprüfungs-Vorrichtung (50, 110) zum Überprüfen eines Gasdetektionsgeräts (100),
wobei das zu überprüfende Gasdetektionsgerät (100)
- eine Messkammer (2),
- einen Detektor (4) und
- eine Strahlungsquelle (1)
umfasst,
wobei die Messkammer (2) dazu ausgestaltet ist, eine zu untersuchende Gasprobe (Gp_{Üb}, Gp_{Ref}) aufzunehmen,
wobei die Strahlungsquelle (1) dazu ausgestaltet ist, Strahlung (eW) in die Messkammer (2) zu emittieren,
wobei das Gasdetektionsgerät (100) so ausgestaltet ist, dass
- wenigstens ein Teil der emittierten Strahlung (eW) mindestens einmal wenigstens einen Teil der Gasprobe (Gp_{Üb}, Gp_{Ref}) in der Messkammer (2) durchdringt und
- die Intensität [S_{Ref}(t), s_{Üb}(t)] der Strahlung (eW) in der Messkammer (2) von einem Eingangssignal [x_{Ref}(t), x_{Üb}(t)] abhängt,
wobei das Eingangssignal [x_{Ref}(t), x_{Üb}(t)] ein Maß für die Intensität der emittierten Strahlung (eW) vor dem Eintritt in die Messkammer (2) ist,
wobei der Detektor (4) dazu ausgestaltet ist, abhängig von der Intensität [S_{Ref}(t), s_{Üb}(t)] der Strahlung (eW) in der Messkammer (2) ein Ausgangssignal [y_{Ref}(t), y_{Üb}(t)] zu generieren,
wobei die Überprüfungs-Vorrichtung (50, 110)
- eine Signalverarbeitungseinheit (50) umfasst und
- wenigstens zeitweise in einer Datenverbindung mit dem Gasdetektionsgerät (100) steht,
wobei die Überprüfungs-Vorrichtung (50, 110) dazu ausgestaltet ist,
sowohl in einem Referenz-Zeitraum (Ref_ZR) als auch in einem vom Referenz-Zeitraum (Ref_ZR) verschiedenen Überprüfungs-Zeitraum (Üb_ZR) jeweils die Schritte auszulösen, dass
- die Strahlungsquelle (1) Strahlung in die Messkammer (2) emittiert,
- als das jeweilige Eingangssignal [x_{Ref}(t), x_{Üb}(t)] ein Maß für die jeweiligen Intensität der emittierten Strahlung (eW) erfasst oder gemessen wird, bevor die Strahlung in die Messkammer (2) eintritt, und
- der Detektor (4) jeweils ein Ausgangssignal [y_{Ref}(t), y_{Üb}(t)] generiert,
wobei das Ausgangssignal [y_{Ref}(t), y_{Üb}(t)] von der Intensität der Strahlung (eW) abhängt und
wobei die gemessene Intensität [s_{Ref}(t), s_{Üb}(t)] auftritt, nachdem die Strahlung (eW) wenigstens einen Teil der Gasprobe (Gp_{Üb}, Gp_{Ref}) in der Messkammer (2) durchdrungen hat,
wobei die Strahlungsquelle (1), die Messkammer (2) und der Detektor (4) im Referenz-Zeitraum (Ref_ZR) intakt sind,
wobei die Signalverarbeitungseinheit (50) dazu ausgestaltet ist, im Zeitbereich oder im Frequenzbereich ein Maß für ein Systemverhalten eines gedachten Systems zu berechnen,
wobei dieses gedachte System mit dem Eingangssignal [x_{Ref}(t), x_{Üb}(t)] angeregt wird und dieses System als Reaktion auf diese Anregung das Ausgangssignal [y_{Ref}(t), y_{Üb}(t)] erzeugt,
wobei die Signalverarbeitungseinheit (50) dazu ausgestaltet ist,
- das Überprüfungs-Systemverhalten, das ist das Systemverhalten des Systems, das im Überprüfungs-Zeitraum (Üb_ZR) mit dem Eingangssignal [x_{Üb}(t)] angeregt wird und als Reaktion das Ausgangssignal [y_{Üb} (t)] erzeugt, mit
- dem Referenz-Systemverhalten, das ist das Systemverhalten des Systems, das im Referenz-Zeitraum (Ref_ZR) mit dem Eingangssignal [x_{Ref}(t)] angeregt wird und als Reaktion das Ausgangssignal [y_{Ref}(t)] erzeugt,
unter Verwendung der beiden Maße [G_{Ref}(S), G_{Üb}(S)] zu vergleichen und
wobei die Signalverarbeitungseinheit (50) weiterhin dazu ausgestaltet ist, abhängig vom Ergebnis des Vergleichs zwischen den beiden Systemverhalten eine Zustands-Information (Zust) über den Zustand des Gasdetektionsgeräts (100) im Überprüfungs-Zeitraum (Üb_ZR) herzuleiten.

12. Mess-Anordnung umfassend
- ein Gasdetektionsgerät (100) und
- eine Überprüfungs-Vorrichtung (50, 110),
wobei das Gasdetektionsgerät (100)
- eine Messkammer (2),
- einen Detektor (4) und
- eine Strahlungsquelle (1)
umfasst,
wobei die Messkammer (2) dazu ausgestaltet ist, eine zu untersuchende Gasprobe (Gp_{Üb}, Gp_{Ref}) aufzunehmen,
wobei die Strahlungsquelle (1) dazu ausgestaltet ist, Strahlung (eW) in die Messkammer (2) zu emittieren,
wobei das Gasdetektionsgerät (100) so ausgestaltet ist, dass
- wenigstens ein Teil der emittierten Strahlung (eW) mindestens einmal die Messkammer (2) durchdringt und
- die Intensität der Strahlung (eW) in der Messkammer (2) von einem Eingangssignal [x_{Ref}(t), x_{Üb}(t)] abhängt,
wobei das Eingangssignal [x_{Ref}(t), x_{Üb}(t)] ein Maß für die Intensität der emittierten Strahlung (eW) vor dem Eintritt in die Messkammer (2) ist,
wobei der Detektor (4) dazu ausgestaltet ist, ein Ausgangssignal [y_{Ref}(t), y_{Üb}(t)] zu generieren,
wobei das Ausgangssignal [y_{Ref}(t), y_{Üb}(t)] von der Intensität der Strahlung (eW) abhängt und
wobei die gemessene Intensität [s_{Ref}(t), s_{Üb}(t)] auftritt, nachdem die Strahlung (eW) wenigstens einen Teil der Gasprobe (Gp_{Üb}, Gp_{Ref}) in der Messkammer (2) durchdrungen hat, und
wobei die Überprüfungs-Vorrichtung (50, 110)
- gemäß Anspruch 11 ausgestaltet ist und
- dazu ausgestaltet ist, das Gasdetektionsgerät (100) zu überprüfen.

## Claims

1. A test method for testing a gas detection device (100),
wherein the gas detection device (100) to be tested comprises
- a measuring chamber (2),
- a detector (4) and
- a radiation source (1),
wherein the measuring chamber (2) is designed to receive a gas sample (Gp_{Üb}, Gp_{Ref}) to be analyzed,
wherein the test method comprises the automatically performed steps, in which
both in a reference period (Ref_ZR) and in a test period (Üb_ZR) different from the reference period (Ref_ZR), in each case
- the radiation source (1) emits radiation (eW) into the measuring chamber (2), in particular electromagnetic radiation,
wherein, as an input signal [x_{Ref}(t), x_{Üb}(t)] in each case, an indicator for the intensity of the emitted radiation (eW) before entry into the measuring chamber (2) is specified or measured,
- at least part of the emitted radiation (eW) penetrates at least part of the gas sample (Gp_{Üb}, Gp_{Ref}) in the measuring chamber (2) at least once so that the radiation (eW) in the measuring chamber (2) has an intensity [s_{Ref}(t), s_{Üb}(t)] that depends on the input signal [x_{Ref}(t), x_{Üb}(t)],
- the detector (4) generates an output signal [y_{Ref}(t), y_{Üb}(t)] depending on the intensity [s_{Ref}(t), s_{Üb}(t)] of the radiation (eW) in the measuring chamber (2),
wherein the measured intensity [s_{Ref}(t), s_{Üb}(t)] occurs after the radiation (eW) has penetrated at least part of the gas sample (Gp_{Üb}, Gp_{Ref}) in the measuring chamber (2) at least once, and
- an indicator [G_{Ref}(s), G_{Üb}(s)] for a system behavior of an intended system is calculated in the time range or in the frequency range,
wherein this intended system is stimulated using the input signal [x_{Ref}(t), x_{Üb}(t)] and this system produces the output signal [y_{Ref}(t), y_{Üb}(t)] in response to this stimulation,
wherein the radiation source (1), the measuring chamber (2) and the detector (4) are intact during the reference period (Ref_ZR) and
wherein the method comprises the further automatically performed steps, in which
- the test system behavior, i.e., the system behavior of the system that is stimulated in the test period (Üb_ZR) using the input signal [x_{Üb}(t)] and produces the output signal [y_{Üb} (t)] in response, is compared with
- the reference system behavior, i.e., the system behavior of the system that is stimulated in the reference period (Ref_ZR) using the input signal [x_{Ref}(t)] and produces the output signal [y_{Ref}(t)] in response,
and
wherein the indicator [G_{Üb}(s)] calculated in the test period (Üb_ZR) for the system behavior and the indicator [G_{Ref}(s)] calculated in the reference period (Ref_ZR) for the system behavior are used for the comparison, and
state information (Zust) regarding the state of the gas detection device (100) in the test period (Üb_ZR) is derived depending on the result of the comparison between the two system behaviors.

2. The test method according to claim 1,
**characterized in that**
both in the reference period (Ref_ZR) and in the test period (Üb_ZR), the step of calculating the indicator [G_{Ref}(s), G_{Üb}(s)] for the system behavior comprises the step, in which
an indicator for the cross-correlation [ρ_{xy,Ref}(t), ρ_{xy,Üb}(t)] between the input signal [x_{Ref}(t), x_{Üb}(t)] and the output signal [y_{Ref}(t), y_{Üb}(t)] is calculated.

3. The test method according to claim 2,
**characterized in that**
both in the reference period (Ref_ZR) and in the test period (Üb_ZR), the step of calculating the indicator [G_{Ref}(s), G_{Üb}(s)] for the system behavior comprises the additional steps, in which
- a transformation of the cross-correlation [ρ_{xy,Ref}(t), ρ_{xy,Üb}(t)] into the frequency range is performed,
in particular by way of a Laplace transformation or a Fourier transformation, and
- the indicator [G_{Ref}(s), G_{Üb}(s)] for the system behavior is determined using the result of the transformation of the cross-correlation [ρ_{xy,Ref}(t), ρ_{xy,Üb}(t)] into the frequency range.

4. The test method according to claim 3,
**characterized in that**
in the two periods (Ref_ZR, Üb_ZR), the steps are performed, in which
- the radiation source (1), in the two periods (Ref_ZR, Üb_ZR), emits the radiation (eW) in each case with an input signal [x_{Ref}(t), x_{Üb}(t)] in the form of a white noise into the measuring chamber (2),
- using the transformation of the cross-correlation [ρ_{xy,Ref}(t), ρ_{xy,Üb}(t)] into the frequency range, the relevant transfer function [G_{Ref}(s), G_{Üb}(s)] of the intended system is calculated upon stimulation by the input signal [x_{Ref}(t), x_{Üb}(t)] and
- the relevant indicator [G_{Ref}(s), G_{Üb}(s)] is calculated for the system behavior using the relevant transfer function.

5. The test method according to any of the preceding claims,
**characterized in that**
the intensity of the emitted radiation (eW) in a frequency band is greater than or equal to a lower threshold and
the measuring chamber (2) is filled with a reference gas sample (Gp_{Ref}) in the reference period (Ref_ZR) and with a test gas sample (Gp_{Üb}) in the test period (Üb_ZR),
the two gas samples (Gp_{Ref}, Gp_{Üb}) attenuating the radiation (eW) in the frequency band to the same extent up to a specified tolerance and
in particular, the relevant chemical composition of the two gas samples (Gp_{Ref}, Gp_{Üb}) differing from one another by a maximum of a specified composition tolerance.

6. The test method according to any of the preceding claims,
**characterized in that**
a target gas quantity having at least one target gas (Zg) to be detected, preferably having a plurality of target gases, is specified, and
the frequency band of the radiation (eW) emitted by the radiation source (1) covers one target gas frequency band for the or each target gas of the target gas quantity,
the target gas (Zg) absorbing radiation (eW) in the relevant target gas frequency band more strongly than a specified lower limit.

7. The test method according to any of the preceding claims,
**characterized in that**
the test method is performed using a signal processing unit (50),
the signal processing unit (50) being spatially separated from the gas detection device (100) and
the test method comprising the additional steps, in which
in the two periods (Ref_ZR, Üb_ZR), the relevant input signal [x_{Ref}(t), x_{Üb}(t)] and the relevant output signal [y_{Ref}(t), y_{Üb}(t)] are transmitted from the gas detection device (100) to the signal processing unit (50) and
the signal processing unit (50) performs the steps of
- calculating the indicators [G_{Ref}(s), G_{Üb}(s)] for the test system behavior and the reference system behavior,
- comparing these with one another and
- deriving the state information (Zust) and
- causing the state information (Zust) to be output in at least one form that is perceivable by a human.

8. The test method according to any of the preceding claims,
**characterized in that**
the gas detection device (100) additionally comprises a reference chamber (3) and a reference detector (5),
the reference chamber (3) being filled with a reference gas (Rg) that is free of the or each target gas,
the radiation source (1) emitting radiation [eW, e_{Ref}(t), e_{Üb}(t)] both into the measuring chamber (2) and into the reference chamber (3), both in the reference period (Ref_ZR) and in the test period (Üb_ZR),
so that emitted radiation [eW, e_{Ref}(t), e_{Üb}(t)] penetrates each of the two chambers (2, 3) at least once,
the two chambers (2, 3) being arranged in parallel with respect to the radiation [eW, e_{Ref}(t), e_{Üb}(t)],
the reference detector (5) producing a time-resolved signal [x_{Ref}(t), x_{Üb}(t)], both in the reference period (Ref_ZR) and in the test period (Üb_ZR), depending in each case on the intensity of emitted radiation (eW) in the reference chamber (3), and
with a system that is stimulated with the signal from the reference detector (5) as the input signal [x_{Ref}(t), x_{Üb}(t)] and produces the output signal [y_{Ref}(t), y_{Üb}(t)] in response to this stimulation being used as the intended system.

9. The test method according to any of the preceding claims,
**characterized in that**
both in the reference period (Ref_ZR) and in the test period (Üb_ZR), the detector (4) in each case produces the output signal [y_{Ref}(t), y_{Üb}(t)] depending on the intensity [s_{Ref}(t), s_{Üb}(t)] of the radiation (eW) in the measuring chamber (2) and on the value of a parameter,
with a plurality of possible values being specified for the parameter and
the method comprising the further automatically performed steps, in which
during the test period (Üb_ZR),
- for each possible value of the parameter, the indicator [G_{Üb}(s)] resulting for this possible parameter value for the test system behavior is calculated and compared with the indicator [G_{Ref}(s)] for the reference system behavior and
- an indicator is determined for the deviation between the test system behavior resulting for this possible parameter value and the reference system behavior [G_{Ref}(s)] and,
a value for the parameter is set and used depending on the deviation indicators for the possible parameter values,
in particular the parameter value is used that leads to the smallest deviation indicator.

10. A measuring method for measuring the concentration of a target gas (Zg)
using a gas detection device (100) which comprises
- a measuring chamber (2),
- a detector (4) and
- a radiation source (1),
wherein the measuring method comprises the automatically performed steps, in which
- the measuring chamber (2) receives a gas sample (Gp_{Üb}) to be analyzed,
- the radiation source (1) emits radiation (eW) into the measuring chamber (2),
- at least part of the emitted radiation (eW) penetrates at least part of the gas sample (Gp_{Üb}) in the measuring chamber (2) at least once so that the radiation (eW) in the measuring chamber (2) has an intensity that depends on the input signal [x_{Üb}(t)] and on the gas sample (Gp_{Üb}),
- the detector (4) generates an output signal [y_{Üb}(t)] depending on the intensity of the radiation (eW) in the measuring chamber (2),
wherein the measured intensity occurs after the radiation (eW) has penetrated at least part of the gas sample in the measuring chamber (2) at least once, and
- the target gas concentration is measured depending on the output signal [y_{Üb}(t)], and
wherein a test method according to any of the preceding claims is performed at least once for the gas detection device (100).

11. A test apparatus (50, 110) for testing a gas detection device (100),
wherein the gas detection device (100) to be tested comprises
- a measuring chamber (2),
- a detector (4) and
- a radiation source (1),
wherein the measuring chamber (2) is designed to receive a gas sample (Gp_{Üb}, Gp_{Ref}) to be analyzed,
wherein the radiation source (1) is designed to emit radiation (eW) into the measuring chamber (2),
wherein the gas detection device (100) is designed such that
- at least part of the emitted radiation (eW) penetrates at least part of the gas sample (Gp_{Üb}, Gp_{Ref}) in the measuring chamber (2) at least once and
- the intensity [s_{Ref}(t), s_{Üb}(t)] of the radiation (eW) in the measuring chamber (2) depends on an input signal [x_{Ref}(t), x_{Üb}(t)],
wherein the input signal [x_{Ref}(t), x_{Üb}(t)] is an indicator for the intensity of the emitted radiation (eW) before it enters the measuring chamber (2),
wherein the detector (4) is designed to generate an output signal [y_{Ref}(t), y_{Üb}(t)] depending on the intensity [s_{Ref}(t), s_{Üb}(t)] of the radiation (eW) in the measuring chamber (2),
wherein the test apparatus (50, 110)
- comprises a signal processing unit (50) and
- is at least temporarily in a data connection with the gas detection device (100),
wherein the test apparatus (50, 110) is designed,
both in a reference period (Ref_ZR) and in a test period (Üb_ZR) that is different from the reference period (Ref_ZR), to trigger the steps, in which
- the radiation source (1) emits radiation into the measuring chamber (2),
- an indicator for the relevant intensity of the emitted radiation (eW) is recorded or measured as the relevant input signal [x_{Ref}(t), x_{Üb}(t)] before the radiation enters into the measuring chamber (2), and
- the detector (4) generates an output signal [y_{Ref}(t), y_{Üb}(t)] in each case,
wherein the output signal [y_{Ref}(t), y_{Üb}(t)] depends on the intensity of the radiation (eW) and
wherein the measured intensity [s_{Ref}(t), s_{Üb}(t)] occurs after the radiation (eW) has penetrated at least part of the gas sample (Gp_{Üb}, Gp_{Ref}) in the measuring chamber (2),
wherein the radiation source (1), the measuring chamber (2) and the detector (4) are intact during the reference period (Ref_ZR),
wherein the signal processing unit (50) is designed to calculate an indicator for a system behavior of an intended system in the time range or in the frequency range,
wherein this intended system is stimulated using the input signal [x_{Ref}(t), x_{Üb}(t)] and this system produces the output signal [y_{Ref}(t), y_{Üb}(t)] in response to this stimulation,
wherein the signal processing unit (50) is designed to
- compare the test system behavior, i.e., the system behavior of the system that is stimulated in the test period (Üb_ZR) using the input signal [x_{Üb}(t)] and produces the output signal [y_{Üb} (t)] in response, with
- the reference system behavior, i.e., the system behavior of the system that is stimulated in the reference period (Ref_ZR) using the input signal [x_{Ref}(t)] and produces the output signal [y_{Ref}(t)] in response,
using the two indicators [G_{Ref}(s), G_{Üb}(s)] and
wherein the signal processing unit (50) is further designed to derive state information (Zust) regarding the state of the gas detection device (100) in the test period (Üb_ZR), depending on the result of the comparison between the two system behaviors.

12. A measuring arrangement comprising
- a gas detection device (100) and
- a test apparatus (50, 110),
wherein the gas detection device (100) comprises
- a measuring chamber (2),
- a detector (4) and
- a radiation source (1),
wherein the measuring chamber (2) is designed to receive a gas sample (Gp_{Üb}, Gp_{Ref}) to be analyzed,
wherein the radiation source (1) is designed to emit radiation (eW) into the measuring chamber (2),
wherein the gas detection device (100) is designed such that
- at least part of the emitted radiation (eW) penetrates the measuring chamber (2) at least once and
- the intensity of the radiation (eW) in the measuring chamber (2) depends on an input signal [x_{Ref}(t), x_{Üb}(t)],
wherein the input signal [x_{Ref}(t), x_{Üb}(t)] is an indicator for the intensity of the emitted radiation (eW) before it enters the measuring chamber (2),
wherein the detector (4) is designed to generate an output signal [y_{Ref}(t), y_{Üb}(t)],
wherein the output signal [y_{Ref}(t), y_{Üb}(t)] depends on the intensity of the radiation (eW) and
wherein the measured intensity [s_{Ref}(t), s_{Üb}(t)] occurs after the radiation (eW) has penetrated at least part of the gas sample (Gp_{Üb}, Gp_{Ref}) in the measuring chamber (2), and
wherein the test apparatus (50, 110)
- is designed according to claim 11 and
- is designed to test the gas detection device (100).

## Revendications

1. Procédé de vérification permettant de vérifier un appareil de détection de gaz (100),
dans lequel l'appareil de détection de gaz (100) à vérifier comprend
- une chambre de mesure (2),
- un détecteur (4) et
- une source de rayonnement (1),
dans lequel la chambre de mesure (2) est conçue pour recevoir un échantillon de gaz (Gp_{Üb}, Gp_{Ref}) à analyser,
dans lequel le procédé de vérification comprend les étapes réalisées automatiquement selon lesquelles
à la fois dans une période de référence (Ref_ZR) et dans une période de vérification (Üb_ZR) différente de la période de référence (Ref_ZR), respectivement
- la source de rayonnement (1) émet un rayonnement (eW) dans la chambre de mesure (2), en particulier un rayonnement électromagnétique,
dans lequel une mesure de l'intensité du rayonnement (eW) émis avant son entrée dans la chambre de mesure (2) est prédéfinie ou mesurée en tant que signal d'entrée [x_{Ref}(t), x_{Üb}(t)] respectif,
- au moins une partie du rayonnement (eW) émis traverse au moins une fois au moins une partie de l'échantillon de gaz (Gp_{Üb}, Gp_{Ref}) dans la chambre de mesure (2), de sorte que le rayonnement (eW) dans la chambre de mesure (2) présente une intensité [s_{Ref}(t), s_{Üb}(t)] dépendant du signal d'entrée [x_{Ref}(t), x_{Üb}(t)],
- le détecteur (4) génère un signal de sortie [y_{Ref}(t), y_{Üb}(t)] en fonction de l'intensité [s_{Ref}(t), s_{Üb}(t)] du rayonnement (eW) dans la chambre de mesure (2),
dans lequel l'intensité [s_{Ref}(t), s_{Üb}(t)] mesurée apparait après que le rayonnement (eW) a traversé au moins une fois au moins une partie de l'échantillon de gaz (Gp_{Üb}, Gp_{Ref}) dans la chambre de mesure (2) et
- une mesure [G_{Ref}(s), G_{Üb}(s)] d'un comportement de système d'un système imaginaire est calculée dans le domaine temporel ou dans le domaine fréquentiel,
dans lequel ledit système imaginaire est excité par le signal d'entrée [x_{Ref}(t), x_{Üb}(t)] et ledit système génère le signal de sortie [y_{Ref}(t), y_{Üb}(t)] en réponse à ladite excitation,
dans lequel la source de rayonnement (1), la chambre de mesure (2) et le détecteur (4) sont intacts pendant la période de référence (Ref_ZR) et
dans lequel le procédé comprend les autres étapes réalisées automatiquement selon lesquelles
- le comportement de système de vérification, lequel est le comportement de système du système qui est excité pendant la période de vérification (Üb_ZR) avec le signal d'entrée [x_{Üb}(t)] et qui génère en réponse le signal de sortie [y_{Üb}(t)], est comparé avec
- le comportement de système de référence, lequel est le comportement de système du système qui est excité pendant la période de référence (Ref_ZR) avec le signal d'entrée [x_{Ref}(t)] et qui génère en réponse le signal de sortie [y_{Ref}(t)],
et
dans lequel sont utilisées pour la comparaison la mesure [G_{Üb}(s)] du comportement de système calculée dans la période de vérification (Üb_ZR) et la mesure [G_{Ref}(s)] du comportement de système calculée dans la période de référence (Ref_ZR), et
des informations d'état (Zust) concernant l'état de l'appareil de détection de gaz (100) dans la période de vérification (Üb_ZR) sont déduites en fonction du résultat de la comparaison entre les deux comportements de système.

2. Procédé de vérification selon la revendication 1,
**caractérisé en ce que**
à la fois dans la période de référence (Ref_ZR) et dans la période de vérification (Üb_ZR), l'étape consistant à calculer la mesure [G_{Ref}(s), G_{Üb}(s)] pour le comportement de système comprend respectivement l'étape selon laquelle
une mesure de la corrélation croisée [ρ_{xy,Ref}(t), ρ_{xy,Üb}(t)] entre le signal d'entrée [x_{Ref}(t), x_{Üb}(t)] et le signal de sortie [y_{Ref}(t), y_{Üb}(t)] est calculée.

3. Procédé de vérification selon la revendication 2,
**caractérisé en ce que**
à la fois dans la période de référence (Ref_ZR) et dans la période de vérification (Üb_ZR), l'étape consistant à calculer la mesure [G_{Ref}(s), G_{Üb}(s)] du comportement de système comprend respectivement les étapes supplémentaires selon lesquelles
- une transformation de la corrélation croisée [ρ_{xy,Ref}(t), ρ_{xy,Üb}(t)] est réalisée dans le domaine fréquentiel,
en particulier par une transformée de Laplace ou une transformée de Fourier, et
- la mesure [G_{Ref}(s), G_{Üb}(s)] du comportement de système est déterminée à l'aide du résultat de la transformation de la corrélation croisée [ρ_{xy,Ref}(t), ρ_{xy,Üb}(t)] dans le domaine fréquentiel.

4. Procédé de vérification selon la revendication 3,
**caractérisé en ce que**
dans les deux périodes (Ref_ZR, Üb_ZR), les étapes sont réalisées, selon lesquelles
- la source de rayonnement (1) émet dans les deux périodes (Ref_ZR, Üb_ZR) respectivement le rayonnement (eW) avec un signal d'entrée [x_{Ref}(t), x_{Üb}(t)] sous la forme d'un bruit blanc dans la chambre de mesure (2),
- à l'aide de la transformation de la corrélation croisée [ρ_{xy,Ref}(t), ρ_{xy,Üb}(t)] dans le domaine fréquentiel, la fonction de transfert [G_{Ref}(s), G_{Üb}(s)] respective du système imaginaire est calculée lors d'une excitation par le signal d'entrée [x_{Ref}(t), x_{Üb}(t)] et
- la mesure [G_{Ref}(s), G_{Üb}(s)] respective du comportement de système est calculée à l'aide de la fonction de transfert respective.

5. Procédé de vérification selon l'une des revendications précédentes, **caractérisé en ce que**
l'intensité du rayonnement (eW) émis dans une bande de fréquences est supérieure ou égale à une valeur seuil inférieure et
la chambre de mesure (2) est remplie d'un échantillon de gaz de référence (Gp_{Ref}) pendant la période de référence (Ref_ZR) et d'un échantillon de gaz de vérification (Gp_{Üb}) pendant la période de vérification (Üb_ZR),
dans lequel les deux échantillons de gaz (Gp_{Ref}, Gp_{Üb}) atténuent le rayonnement (eW) dans la bande de fréquences de la même manière, à une tolérance prédéfinie près, et
dans lequel, en particulier, les compositions chimiques respective des deux échantillons de gaz (Gp_{Ref}, Gp_{Üb}) diffèrent l'une de l'autre au maximum d'une tolérance de composition prédéfinie.

6. Procédé de vérification selon l'une des revendications précédentes, **caractérisé en ce que**
une quantité de gaz cible est prédéfinie avec au moins un gaz cible (Zg) à détecter, de préférence avec plusieurs gaz cibles, et
la bande de fréquences du rayonnement (eW) émis par la source de rayonnement (1) couvre respectivement une bande de fréquences de gaz cible pour le ou chaque gaz cible de la quantité de gaz cible,
dans lequel le gaz cible (Zg) absorbe le rayonnement (eW) dans la bande de fréquences de gaz cible respective plus fortement qu'une limitation inférieure prédéfinie.

7. Procédé de vérification selon l'une des revendications précédentes,
**caractérisé en ce que**
le procédé de vérification est réalisé à l'aide d'une unité de traitement de signaux (50),
dans lequel l'unité de traitement de signaux (50) est espacée spatialement de l'appareil de détection de gaz (100) et
dans lequel le procédé de vérification comprend les étapes supplémentaires selon lesquelles
dans les deux périodes (Ref_ZR, Üb_ZR), le signal d'entrée [x_{Ref}(t), x_{Üb}(t)] respectif et le signal de sortie [y_{Ref}(t), y_{Üb}(t)] respectif sont transmis de l'appareil de détection de gaz (100) à l'unité de traitement de signaux (50) et
l'unité de traitement de signaux (50) réalise les étapes pour
- calculer les mesures [G_{Ref}(s), G_{Üb}(s)] du comportement de système de vérification et du comportement de système de référence,
- les comparer entre elles et
- déduire les informations d'état (Zust) et
- s'assurer que les informations d'état (Zust) sont délivrées en sortie sous au moins une forme perceptible par un être humain.

8. Procédé de vérification selon l'une des revendications précédentes,
**caractérisé en ce que**
l'appareil de détection de gaz (100) comprend en outre une chambre de référence (3) et un détecteur de référence (5),
dans lequel la chambre de référence (3) est remplie d'un gaz de référence (Rg) qui est exempt du ou de chaque gaz cible,
dans lequel la source de rayonnement (1) émet un rayonnement [eW, e_{Ref}(t), e_{Üb}(t)] à la fois dans la chambre de mesure (2) et dans la chambre de référence (3) pendant la période de référence (Ref_ZR) et la période de vérification (Üb_ZR),
de sorte que le rayonnement [eW, e_{Ref}(t), e_{Üb}(t)] émis traverse les deux chambres (2, 3) respectivement au moins une fois,
dans lequel les deux chambres (2, 3) sont disposées parallèlement par rapport au rayonnement [eW, e_{Ref}(t), e_{Üb}(t)],
dans lequel le détecteur de référence (5) génère un signal [_{xRef}(t), _{xÜb}(t)] résolu dans le temps à la fois pendant la période de référence (Ref_ZR) et pendant la période de vérification (Üb_ZR), respectivement en fonction de l'intensité du rayonnement (eW) émis dans la chambre de référence (3), et
dans lequel est utilisé comme système imaginaire un système qui est excité avec le signal provenant du détecteur de référence (5) en tant que signal d'entrée [x_{Ref}(t), x_{Üb}(t)] et qui génère le signal de sortie [y_{Ref}(t), y_{Üb}(t)] en réponse à ladite excitation.

9. Procédé de vérification selon l'une des revendications précédentes,
**caractérisé en ce que**
à la fois pendant la période de référence (Ref_ZR) et pendant la période de vérification (Üb_ZR), le détecteur (4) génère respectivement le signal de sortie [y_{Ref}(t), y_{Üb}(t)] en fonction de l'intensité [s_{Ref}(t), s_{Üb}(t)] du rayonnement (eW) dans la chambre de mesure (2) et de la valeur d'un paramètre,
dans lequel plusieurs valeurs possibles sont prédéfinies pour le paramètre et
dans lequel le procédé comprend les autres étapes réalisées automatiquement selon lesquelles
pendant la période de vérification (Üb_ZR)
- pour chaque valeur possible du paramètre, la mesure [G_{Üb}(s)] du comportement de système de vérification résultant de ladite valeur possible du paramètre est calculée et comparée avec la mesure [G_{Ref}(s)] du comportement de système de référence et
- une mesure de l'écart entre le comportement de système de vérification résultant de ladite valeur possible du paramètre et le comportement de système de référence [G_{Ref}(s)] est déterminée et
en fonction des mesures d'écart des valeurs possibles du paramètre, une valeur est définie et utilisée pour le paramètre,
en particulier, la valeur de paramètre qui conduit à la mesure d'écart la plus faible est utilisée.

10. Procédé de mesure permettant la mesure de la concentration d'un gaz cible (Zg)
à l'aide d'un appareil de détection de gaz (100) qui comprend
- une chambre de mesure (2),
- un détecteur (4) et
- une source de rayonnement (1),
dans lequel le procédé de mesure comprend les étapes réalisées automatiquement selon lesquelles
- la chambre de mesure (2) reçoit un échantillon de gaz (Gp_{Üb}) à analyser,
- la source de rayonnement (1) émet un rayonnement (eW) dans la chambre de mesure (2),
- au moins une partie du rayonnement (eW) émis traverse au moins une fois au moins une partie de l'échantillon de gaz (Gp_{Üb}) dans la chambre de mesure (2), de sorte que le rayonnement (eW) dans la chambre de mesure (2) présente une intensité qui dépend du signal d'entrée [x_{Üb}(t)] et de l'échantillon de gaz (Gp_{Üb}),
- le détecteur (4) génère un signal de sortie [y_{Üb}(t)] en fonction de l'intensité du rayonnement (eW) dans la chambre de mesure (2),
dans lequel l'intensité mesurée apparait après que le rayonnement (eW) a traversé au moins une fois au moins une partie de l'échantillon de gaz dans la chambre de mesure (2), et
- la concentration de gaz cible est mesurée en fonction du signal de sortie [y_{Üb}(t)], et
dans lequel un procédé de vérification selon l'une des revendications précédentes est réalisé au moins une fois pour l'appareil de détection de gaz (100).

11. Dispositif de vérification (50, 110) permettant de vérifier un appareil de détection de gaz (100),
dans lequel l'appareil de détection de gaz (100) à vérifier comprend
- une chambre de mesure (2),
- un détecteur (4) et
- une source de rayonnement (1),
dans lequel la chambre de mesure (2) est conçue pour recevoir un échantillon de gaz (Gp_{Üb}, Gp_{Ref}) à analyser,
dans lequel la source de rayonnement (1) est configurée pour émettre un rayonnement (eW) dans la chambre de mesure (2),
dans lequel l'appareil de détection de gaz (100) est configuré de sorte que
- au moins une partie du rayonnement (eW) émis traverse au moins une fois au moins une partie de l'échantillon de gaz (Gp_{Üb}, Gp_{Ref}) dans la chambre de mesure (2) et
- l'intensité [s_{Ref}(t), s_{Üb}(t)] du rayonnement (eW) dans la chambre de mesure (2) dépend d'un signal d'entrée [x_{Ref}(t), x_{Üb}(t)],
dans lequel le signal d'entrée [x_{Ref}(t), x_{Üb}(t)] est une mesure de l'intensité du rayonnement (eW) émis avant son entrée dans la chambre de mesure (2),
dans lequel le détecteur (4) est configuré pour générer un signal de sortie [y_{Ref}(t), y_{Üb}(t)] en fonction de l'intensité [s_{Ref}(t), s_{Üb}(t)] du rayonnement (eW) dans la chambre de mesure (2),
dans lequel le dispositif de vérification (50, 110)
- comprend une unité de traitement de signaux (50) et
- est au moins temporairement en connexion de données avec l'appareil de détection de gaz (100),
dans lequel le dispositif de vérification (50, 110) est configuré pour
déclencher, à la fois pendant une période de référence (Ref_ZR) et pendant une période de vérification (Üb_ZR) différente de la période de référence (Ref_ZR), respectivement les étapes selon lesquelles
- la source de rayonnement (1) émet un rayonnement dans la chambre de mesure (2),
- une mesure de l'intensité respective du rayonnement (eW) émis est détectée ou mesurée en tant que signal d'entrée [x_{Ref}(t), x_{Üb}(t)] respectif avant que le rayonnement n'entre dans la chambre de mesure (2), et
- le détecteur (4) génère respectivement un signal de sortie [y_{Ref}(t), y_{Üb}(t)],
dans lequel le signal de sortie [y_{Ref}(t), y_{Üb}(t)] dépend de l'intensité du rayonnement (eW) et
dans lequel l'intensité [s_{Ref}(t), s_{Üb}(t)] mesurée apparait après que le rayonnement (eW) a traversé au moins une partie de l'échantillon de gaz (Gp_{Üb}, Gp_{Ref}) dans la chambre de mesure (2),
dans lequel la source de rayonnement (1), la chambre de mesure (2) et le détecteur (4) sont intacts pendant la période de référence (Ref_ZR),
dans lequel l'unité de traitement de signaux (50) est configurée pour calculer, dans le domaine temporel ou dans le domaine fréquentiel, une mesure d'un comportement de système d'un système imaginaire,
dans lequel ledit système imaginaire est excité par le signal d'entrée [x_{Ref}(t), x_{Üb}(t)] et ledit système génère le signal de sortie [y_{Ref}(t), y_{Üb}(t)] en réponse à ladite excitation,
dans lequel l'unité de traitement de signaux (50) est configurée pour comparer
- le comportement de système de vérification, lequel est le comportement de système du système qui est excité pendant la période de vérification (Üb_ZR) avec le signal d'entrée [x_{Üb}(t)] et qui génère en réponse le signal de sortie [y_{Üb} (t)], avec
- le comportement de système de référence, lequel est le comportement de système du système qui est excité pendant la période de référence (Ref_ZR) avec le signal d'entrée [x_{Ref}(t)] et qui génère en réponse le signal de sortie [y_{Ref}(t)],
à l'aide des deux mesures [G_{Ref}(s), G_{Üb}(s)] et
dans lequel l'unité de traitement de signaux (50) est en outre configurée pour déduire, en fonction du résultat de la comparaison entre les deux comportements de système, des informations d'état (Zust) concernant l'état de l'appareil de détection de gaz (100) pendant la période de vérification (Üb_ZR).

12. Agencement de mesure comprenant
- un appareil de détection de gaz (100) et
- un dispositif de vérification (50, 110),
dans lequel l'appareil de détection de gaz (100) comprend
- une chambre de mesure (2),
- un détecteur (4) et
- une source de rayonnement (1),
dans lequel la chambre de mesure (2) est conçue pour recevoir un échantillon de gaz (Gp_{Üb}, Gp_{Ref}) à analyser,
dans lequel la source de rayonnement (1) est configurée pour émettre un rayonnement (eW) dans la chambre de mesure (2),
dans lequel l'appareil de détection de gaz (100) est configuré de sorte que
- au moins une partie du rayonnement (eW) émis traverse au moins une fois la chambre de mesure (2) et
- l'intensité du rayonnement (eW) dans la chambre de mesure (2) dépend d'un signal d'entrée [x_{Ref}(t), x_{Üb}(t)],
dans lequel le signal d'entrée [x_{Ref}(t), x_{Üb}(t)] est une mesure de l'intensité du rayonnement (eW) émis avant son entrée dans la chambre de mesure (2),
dans lequel le détecteur (4) est configuré pour générer un signal de sortie [y_{Ref}(t), y_{Üb}(t)],
dans lequel le signal de sortie [y_{Ref}(t), y_{Üb}(t)] dépend de l'intensité du rayonnement (eW) et
dans lequel l'intensité [s_{Ref}(t), s_{Üb}(t)] mesurée apparait après que le rayonnement (eW) a traversé au moins une partie de l'échantillon de gaz (Gp_{Üb}, Gp_{Ref}) dans la chambre de mesure (2), et
dans lequel le dispositif de vérification (50, 110)
- est conçu selon la revendication 11 et
- est configuré pour vérifier l'appareil de détection de gaz (100).
